# EUROPÄISCHE PATENTSCHRIFT

(11) **EP 2 374 795 B1**
(45) Veröffentlichungstag und Bekanntmachung des Hinweises auf die Patenterteilung: **16.03.2016**
(21) Anmeldenummer: 11158120.3
(22) Anmeldetag: 02.07.2004
(51) Int. Cl.: C07D 215/12, G01N 33/58, C07D 215/14, C07D 215/20, C07D 215/38, C07D 401/12, C07D 405/12, C07D 221/18, C07D 471/04, C07D 491/04, C07D 491/14, C07D 491/22, C07D 413/12, C07D 498/04, C07D 498/14

(54) **Sulfonsäurederivate polycyclischer Farbstoffe für analytische Anwendungen**
Sulphonic acid derivatives of polycyclic dyes used for analytical applications
Dérivés d'acide sulfonique de colorants polycycliques pour applications analytiques

(30) Priorität: 02.07.2003 DE 10329860
(43) Veröffentlichungstag der Anmeldung: 12.10.2011
(62) Teilanmeldung aus: 04763083.5
(73) Patentinhaber: ATTO-TEC GmbH, 57076 Siegen (DE)
(72) Erfinder: Zilles, Alexander, 55490, Mengerschied (DE); Arden-Jacob, Jutta, 90513, Zirndorf (DE); Drexhage, Karl-Heinz, 57076, Siegen (DE); Kemnitzer, Norbert Uwe, 57250, Netphen (DE); Hamers-Schneider, Monika, 57258, Freudenberg (DE)
(74) Vertreter: Weickmann & Weickmann

(56) Entgegenhaltungen:
- WO-A-99/15517

## Beschreibung

Die Erfindung betrifft Sulfonsäuregruppen-enthaltende Farbstoffe. Die erfindungsgemäßen Farbstoffe finden insbesondere Anwendung zur Markierung von Analyten, beispielsweise zur Markierung von Biomolekülen.

Farbstoffe aus der Klasse der Cumarin-, der Xanthen-, der Oxazinfarbstoffe sowie verwandte Derivate werden aufgrund ihrer sehr guten spektralen Eigenschaften, insbesondere ihrer Fluoreszenz, bevorzugt in der chemischen, biologischen und medizinischen Analytik als Markierungsgruppen eingesetzt (J. Slavik, Fluorescent Probes in Cellular and Molecular Biology, CRC Press, Boca Raton, Ann Arbor, London, Tokyo, 1994). Eine große Anzahl von langwelligen Xanthen-Farbstoffen (Rhodamine etc.) werden beschrieben in EP 0567622 B1. Markierungsfarbstoffe aus der Klasse der Oxazine werden offenbart in EP 0747447 B1. Sulfonsäurederivate von Xanthen-Farbstoffen sind beschrieben in WO 99/15517. Farbstoffe aus den Klassen der Carbopyronine und Amidopyrylium-Farbstoffe werden beschrieben in WO 00/64986 und WO 00/64987. Dabei spielen vor allem Farbstoffe mit einer hohen Fluoreszenzquantenausbeute eine wichtige Rolle, da über die Fluoreszenz ein sehr empfindlicher Nachweis des markierten Analyten möglich ist. Aber auch nicht-fluoreszierende Derivate gewinnen zunehmend als Quencher in speziellen Verfahren Bedeutung.

Zur Verwendung als Markierungsgruppen für Analyte ist neben einer einfachen und zuverlässigen Nachweisbarkeit eine gute Löslichkeit, z.B. in wässrigen Systemen, erforderlich. In anderen Fällen ist, gerade umgekehrt, gute Löslichkeit in unpolarer Umgebung, z.B. Zellmembranen, erwünscht.

Von entscheidender Bedeutung für die Anwendbarkeit als Markierungsgruppen für Analyte oder in ähnlichen Verfahren sind also je nach den konkreten Bedingungen bestimmte spezifische Eigenschaften, über die der Fluorophor zusätzlich zu seinen optischen Eigenschaften verfügen sollte. Diese sind bei den Farbstoffen der genannten Patente entweder gar nicht oder, wenn überhaupt, nur sehr umständlich durch Farbstoffsynthese unter Verwendung entsprechender Edukte zu erreichen.

Eine Aufgabe der vorliegenden Erfindung bestand deshalb darin, bestimmte physikalische und/oder chemische Eigenschaften - etwa die Löslichkeit in bestimmten Medien, die Tendenz zu unspezifischen Wechselwirkungen mit Substraten und/oder Gefäßwänden oder Kopplungsfähigkeit . - dieser Farbstoffe in einfacher Weise - auch nachträglich - zu modifizieren, ohne die sehr guten spektralen Eigenschaften der Farbstoffe wesentlich zu verändern.

Die Erfindung betrifft polycyclische Farbstoffe der allgemeinen Formel II", V"-VII" 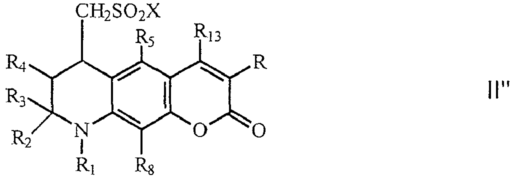 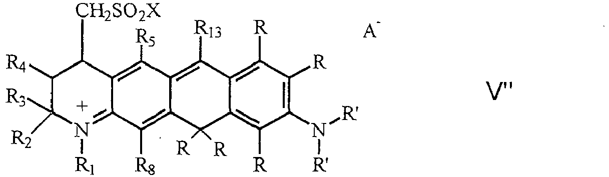 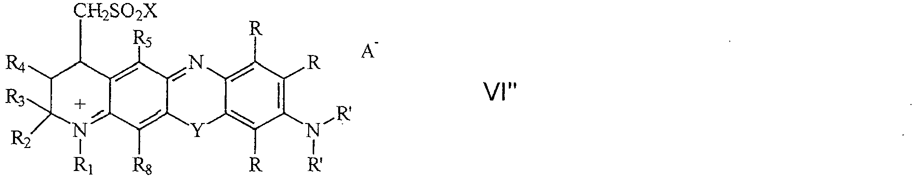 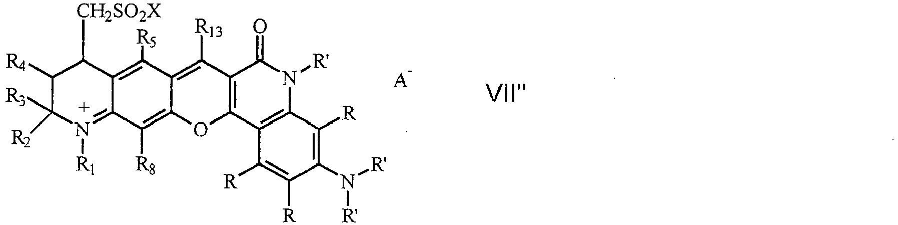 worin
R₁ Wasserstoff oder eine Kohlenwasserstoffgruppe mit 1-20 C-Atomen bedeutet, wobei die Kohlenwasserstoffgruppe gegebenenfalls ein oder mehrere Heteroatome oder/und einen oder mehrere Substituenten enthalten kann,
R₂, R₃, R₄, R₅, R₆, R₇ und R₈ bei jedem Auftreten unabhängig voneinander Wasserstoff, Halogen, eine Hydroxy-, Amino-, Sulfo-, Carboxy- oder Aldehydgruppe oder eine Kohlenwasserstoffgruppe mit 1-20 C-Atomen bedeutet, wobei die Kohlenwasserstoffgruppe gegebenenfalls ein oder mehrere Heteroatome oder/und einen oder mehrere Substituenten enthalten kann, oder die Reste R₁ und R₈ zusammen ein Ringsystem bilden,
R' Wasserstoff oder eine Kohlenwasserstoffgruppe mit 1-20 C-Atomen bedeutet, wobei die Kohlenwasserstoffgruppe gegebenenfalls ein oder mehrere Heteroatome oder/und einen oder mehrere Substituenten enthalten kann,
R bei jedem Auftreten unabhängig voneinander Wasserstoff, Halogen, eine Hydroxy-, Amino-, Sulfo-, Carboxy- oder Aldehydgruppe oder eine Kohlenwasserstoffgruppe mit 1-20 C-Atomen bedeutet, wobei die Kohlenwasserstoffgruppe gegebenenfalls ein oder mehrere Heteroatome oder/und einen oder mehrere Substituenten enthalten kann, oder die Reste R' und R zusammen ein Ringsystem bilden, das eine oder mehrere Mehrfachbindungen enthalten kann,
R₁₃ bei jedem Auftreten unabhängig voneinander Wasserstoff oder eine Kohlenwasserstoffgruppe mit 1-20 C-Atomen bedeutet, wobei die Kohlenwasserstoffgruppe gegebenenfalls ein oder mehrere Heteroatome oder/und einen oder mehrere Substituenten enthalten kann, wobei R₁₃ insbesondere Wasserstoff, Aryl, Carboxyphenyl, Alkyl, Perfluoralkyl, Cycloalkyl, Pyridyl oder Carboxypyridyl) darstellt,
X OH, Halogen, -O-R₉, -S-R₁₀ oder -NR₁₁R₁₂ bedeutet, wobei R₉, R₁₀, R₁₁ und R₁₂ jeweils unabhängig voneinander Wasserstoff oder einen C1 bis C20 Kohlenwasserstoffrest bedeuten, welcher gegebenenfalls ein oder mehrere Heteroatome oder einen oder mehrere Substituenten enthalten kann,
Y in Formel VI" O, S oder C(R)₂ darstellt, und
A- ein Anion bedeutet.

Ferner betrifft die Erfindung ein Verfahren zur Herstellung von polycyclischen Farbstoffen dadurch gekennzeichnet, dass man Verbindungen der Formeln II', V' - VII' 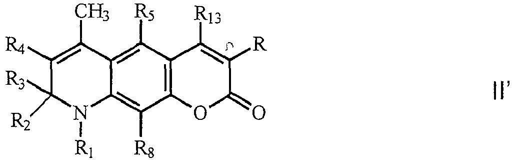 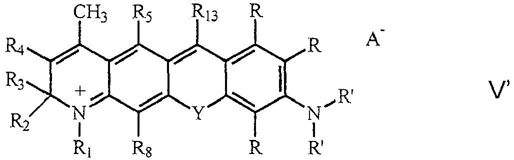 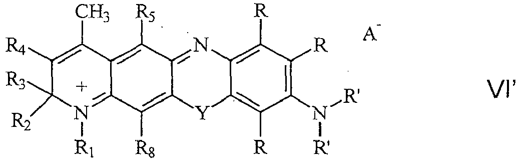 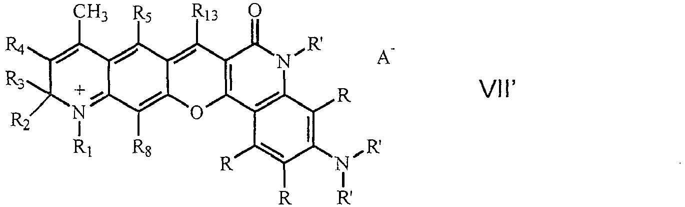 zu entsprechenden Verbindungen der allgemeinen Formeln II, V - VII 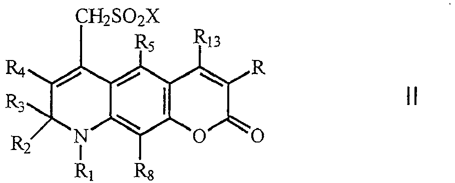 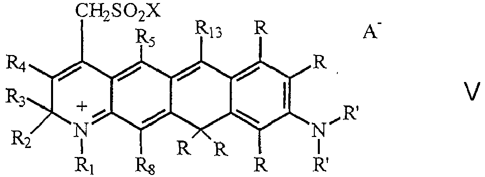 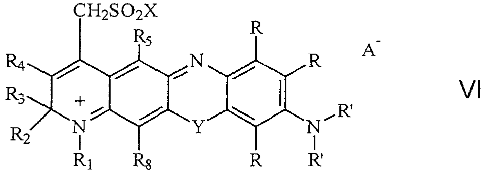 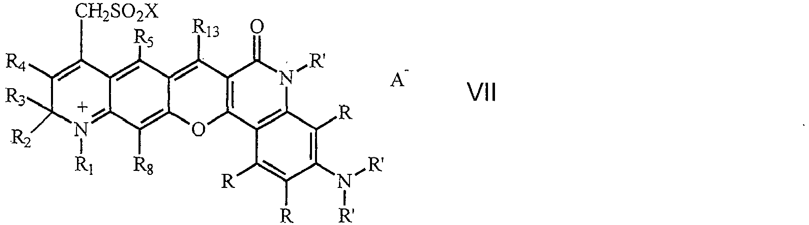 sulfoniert, und die Verbindungen der allgemeinen Formeln II, V - VII anschließend zu entsprechenden Tetrahydrochinolinen der allgemeinen Formeln II", V" - VII" hydriert.

Hierin beschrieben wird die Darstellung von Verbindungen der allgemeinen Formeln I a und I b

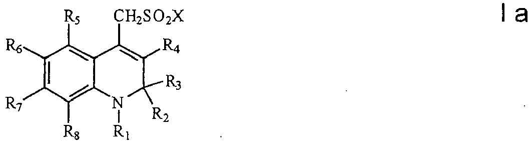

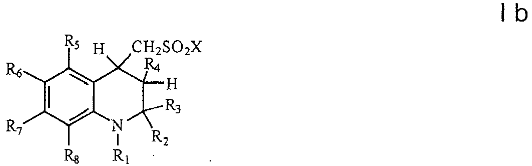

Sowie ihre Verwendung zur Darstellung von Farbstoffen, die z.B. als Markierungsgruppen in einem Verfahren zum Nachweis von Analyten eingesetzt werden können, wobei
R₁ Wasserstoff oder eine gesättigte oder ungesättigte, geradkettige, verzweigte oder cyclische Kohlenwasserstoffgruppe mit bis zu 20 C-Atomen, z.B. Polyether, Phenyl, Phenylalkyl mit 1-3 C-Atomen in der Kette bedeuten, wobei die Kohlenwasserstoffgruppen gegebenenfalls Heteroatome, wie Sauerstoff-, Schwefel- oder Stickstoffatome, oder/und einen oder mehrere Substituenten, vorzugsweise ausgewählt aus Halogenen, Hydroxy-, Amino-, Sulfo-, Phospho-, Carboxy-, Carbonyl-, Alkoxy- oder/und Alkoxycarbonylgruppen, enthalten können,
R₂, R₃, R₄, R₅, R₆, R₇ und R₈ jeweils unabhängig Wasserstoff, Halogen, eine Hydroxy-, Amino-, Sulfo- oder Carboxy- oder Aldehydgruppe oder eine gesättigte oder ungesättigte, geradkettige, verzweigte oder cyclische Kohlenwasserstoffgruppe mit bis zu 20 C-Atomen bedeuten, wobei die Kohlenwasserstoffgruppen Alkyl-, Alkenyl-, Alkinyl-, Cycloalkyl-, Aryl, insbesondere Phenyl-, oder/und Heteroarylreste umfassen und gegebenenfalls auch Heteroatome, wie Sauerstoff-, Schwefel- oder Stickstoffatome, oder/und mehrere Substituenten, vorzugsweise ausgewählt aus Halogenen, Hydroxy-, Amino-, Phospho-, Sulfo-, Carboxy-, Carbonyl-, Alkoxy- oder/und Alkoxycarbonylgruppen, enthalten können, oder der Rest R₈ mit R₁ ein Ringsystem bilden, das eine oder mehrere Mehrfachbindungen enthalten kann, und
X Halogen, eine Hydroxy-, Alkoxy-, Alkylthio- oder Aminogruppe bedeuten, wobei die gegebenenfalls vorhandenen Kohlenwasserstoffreste dieser Gruppen insbesondere Alkyl-, Alkenyl-, Alkinyl-, Cycloalkyl-, Aryl, insbesondere Phenyl-, oder/und Heteroarylreste umfassen und gegebenenfalls auch Heteroatome, wie Sauerstoff-, Schwefel- oder Stickstoffatome, oder/und mehrere Substituenten, vorzugsweise ausgewählt aus Halogenen, Hydroxy-, Amino-, Phospho-, Sulfo-, Carboxy-, Carbonyl-, Alkoxy- oder/und Alkoxycarbonylgruppen, enthalten können.

Bei bevorzugten Ausführungsformen bedeutet R₁ einen Aryl- oder Alkylrest, R₂ und R₃ jeweils Methyl und R₄ Wasserstoff.

In weiteren besonders bevorzugten Formen bedeutet R₁ einen Aryl- oder Alkylrest, R₂ und R₃ jeweils Methyl, R₄ Wasserstoff und R₇ eine Hydroxy- oder Methoxygruppe.

In noch weiteren besonders bevorzugten Formen bedeutet R₁ einen Aryl- oder Alkylrest, R₂ und R₃ jeweils Methyl, R₄ Wasserstoff und R₆ eine Nitroso-, Formyl- oder eine Hydroxymethylgruppe.

In einer weiteren Verbindungsklasse sind R₁ mit R₈ verbrückt und bilden ein Ringsystem, insbesondere einen 5- oder 6-gliedrigen Ring.

Die vorzugsweise verwendeten bzw. dargestellten Farbstoffe sind Vertreter der nachfolgend zusammengestellten Klassen - aber nicht auf diese Klassen beschränkt- der allgemeinen Formeln II - VII. Bei Farbstoffen der Klassen III und V - VII, die über ein symmetrisches chromophores System verfügen, ist es erfindungsgemäß durch Wahl der Edukte möglich, zwei - gleiche oder verschiedene-SO₂X-Gruppen einzuführen. Durch nachträgliche Hydrierung ist es in gleicher Weise wie bei den Edukten I möglich, entsprechende Farbstoffe mit Tetrahydrochinolin-Endgruppen herzustellen.

| | | |
|---|---|---|
| 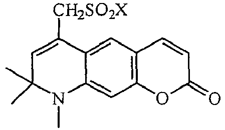 | II | Cumarine |
| 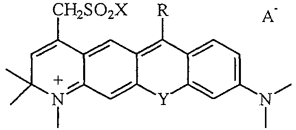 | III | Xanthenfarbstoffe |
| | | Y=O, S, Se |
| 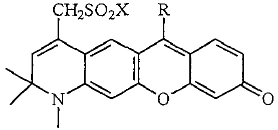 | IV | Rhodol-Farbstoffe |
| 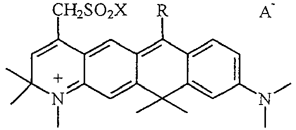 | V | Carbopyronine |
| 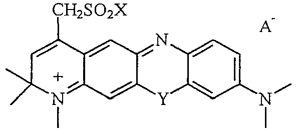 | VI | Azin-Farbstoffe |
| | | Y = O (Oxazine), S (Thiazine), CR₂ (Carbazine) |
| 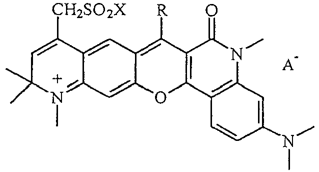 | VII | Amidopyryliumfarbstoffe |

wobei R insbesondere = H, (subst.) Aryl, (subst.) Carboxyphenyl, (subst.) Alkyl, (subst.) Cycloalkyl, (subst.) Pyridyl, (subst.) Carboxypyridyl etc. Erfindungsgemäß können entweder entsprechende Synthesevorstufen oder die eigentlichen Farbstoffe sulfoniert und anschließend die Sulfonsäuregruppe(n) derivatisiert werden. Dazu wird die Sulfonsäure oder ihr Salz bevorzugt in ein Sulfochlorid überführt und dieses anschließend mit einem Nucleophil (z.B. Amino- oder Mercaptogruppe) umgesetzt. Die Sulfonierung gelingt z.B. in konzentrierter Schwefelsäure, gegebenenfalls im Gemisch mit Oleum. Die Sulfonsäurederivate sowohl der Vorstufen als auch der Farbstoffe lassen sich in einfacher Weise mit Phosphoroxychlorid, Phosphorpentachlorid oder Thionylchlorid bevorzugt im Gemisch mit Dimethylformamid zum Sulfochlorid umsetzen. Überraschenderweise können die Sulfochloride in fast allen Fällen problemlos isoliert werden. Die Sulfochloride reagieren mit verschiedenen Nucleophilen, vorzugsweise mit primären oder sekundären Aminen, zu entsprechenden Sulfonamid-Derivaten. Die physikalischen und/oder chemischen Eigenschaften der so erhaltenen Verbindungen hängen dabei auch ganz entscheidend von der Natur des eingesetzten Amins ab, wie später noch ausführlicher - auch anhand von Beispielen - erläutert wird. Dabei wird eine erstaunlich große Vielfalt von pH-stabilen Sulfonamid-Derivaten erhalten. Die erfindungsmäßige Art der Sulfonamid-Verknüpfung sorgt für eine räumliche Separation von Substituent und Chromophor und verhindert dadurch einen nachteiligen Einfluss auf die Fluoreszenz-Effizienz. Die Farbstoffsulfochloride können auch - ohne vorherige Derivatisierung - direkt zur Kopplung an nucleophile (NH₂ etc. Gruppen) Gruppen von Analytmolekülen verwendet werden.

Der Begriff Heteroatome, wie hierin verwendet, umfasst insbesondere Sauerstoff-, Schwefel- und Stickstoffatome.

Der Begriff Substituenten umfasst, soweit nicht anders angegeben, insbesondere Halogen, Hydroxy-, Amino-, Sulfo-, Phospho-, Carboxy-, Alkoxy- sowie Alkoxycarbonylgruppen mit, soweit vorhanden, 1-10 C-Atomen.

Zur Darstellung der erfindungsgemäßen Verbindungen werden folgende allgemeine Verfahren angewendet. Es können entweder die Vorstufen (Punkt 1) oder die fertigen Farbstoffe (Punkt 2) derivatisiert werden.

### 1. Synthese entsprechender Vorstufen

Ausgehend von einem nach literaturbekannten Methoden, z.B. durch die Kondensation eines Anilinderivats mit Mesityloxid und anschließende N-Alkylierung, dargestellten 7-Alkoxy-2,2,4-trimethyl-1,2-dihydrochinolin wird folgendermaßen vorgegangen:

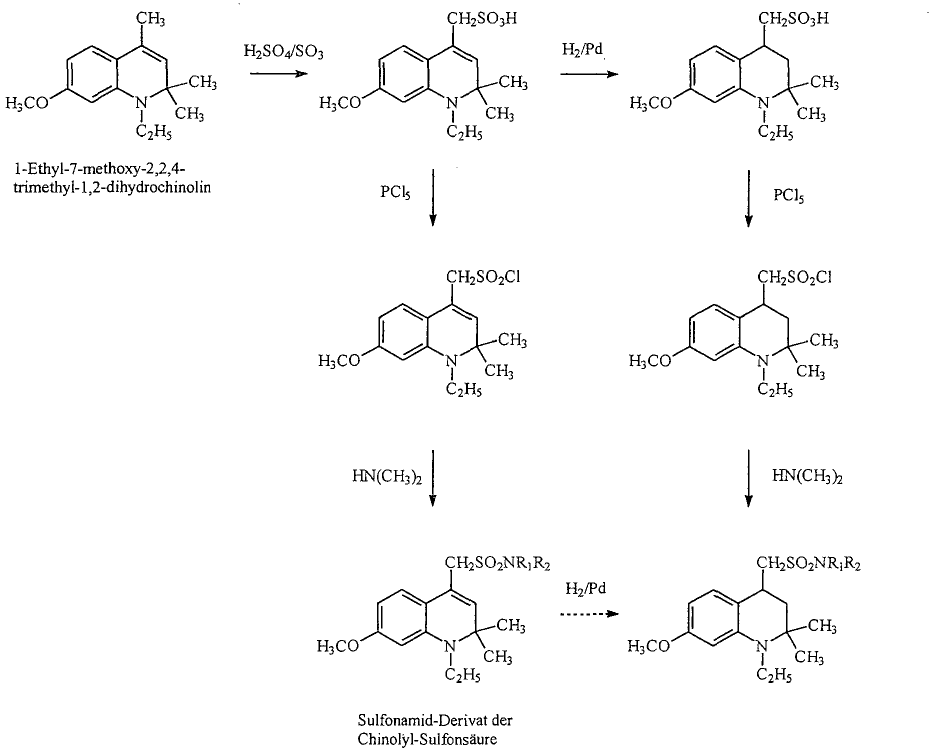

### Abbildung 1:

Beispielhafte Darstellung hierin beschriebener Verbindungen. Es wird ausgehend von 1-Ethyl-7-methoxy-2,2,4-trimethyl-1,2-dihydrochinolin über die Zwischenstufen der Sulfonsäure und des Sulfochlorids schließlich ein Sulfonamid-Derivat erhalten. Durch intermediäre Hydrierung lässt sich das entsprechende 1,2,3,4-Tetrahydrochinolin-Derivat erhalten.

Durch Verrühren des Dihydrochinolins mit einem Gemisch aus konzentrierter Schwefelsäure und Oleum bei Raumtemperatur erhält man ein an der 4-Methylgruppe sulfoniertes Produkt. Diese Sulfonsäure oder ihr Salz kann leicht in das Sulfochlorid überführt werden. Dazu wird die sulfonierte Verbindung bevorzugt bei Raumtemperatur in Benzol mit Phosphorpentachlorid umgesetzt. Allgemein verwendet man für die Darstellung des Sulfochlorids gebräuchliche Reagenzien wie Phosphoroxychlorid, Phosphorpentachlorid oder Thionylchlorid. Die Reaktion wird in einem wasserfreien inerten Lösungsmittel, vorzugsweise Benzol, ausgeführt.

Das Sulfochlorid kann durch einfache Umsetzung mit fast beliebigen primären und sekundären Aminen in ein entsprechendes Sulfonamid überführt werden. Umsetzungen des Sulfochlorids mit anderen Nucleophilen (Thiole, Alkohole etc.) sind ebenfalls möglich. Gegebenenfalls kann das erhaltene stabile Sulfonamid weiter derivatisiert werden (Verseifung von Carbonestergruppen o.ä.).

Das 1,2-Dihydrochinolin lässt sich z.B. auf der Stufe der Sulfonsäure palladiumkatalysiert mit Wasserstoff hydrieren und so in ein 1,2,3,4-Tetrahydrochinolin überführen. Dieses hydrierte Chinolin kann anschließend dem gleichen Syntheseweg unterworfen werden (siehe Abbildung 1).

Mit den so erhaltenen Sulfonsäuren, ihren Salzen oder bevorzugt den Sulfonamid-Derivaten werden die nachfolgenden Farbstoffsynthesen nach den dem Fachmann bekannten Darstellungsmethoden durchgeführt:
Symmetrische Rhodaminfarbstoffe lassen sich z.B. durch die Kondensation des beschriebenen Sulfonamid-Derivats mit Phthalsäureanhydrid erhalten. Andere Xanthenfarbstoffe, wie z.B. Trifluormethyl-, Pyronin- oder Rosaminfarbstoffe, erhält man durch Kondensation mit den entsprechenden Säureanhydriden (Trifluoressigsäureanhydrid) oder Aldehyden (Benzaldehyd etc.).

Unsymmetrische Farbstoffe erhält man im Falle der Rhodamine z.B. durch die Umsetzung von Phthalsäureanhydrid mit einem Aminophenol oder hydriertem 7-Hydroxy- bzw. 7-Methoxychinolin und die anschließende Umsetzung des gebildeten Benzophenon-Derivats, z.B. mit einem zweiten Dihydro- oder Tetrahydrochinolin, wobei jetzt etwa ein erfindungsgemäßes Sulfonamid-Derivat zum Einsatz kommen kann.

Die Vertreter anderer Farbstoffklassen, wie z.B. Cumarine, Carbopyronine, Amidopyryliumfarbstoffe, Oxazine usw., lassen sich mit den Sulfonsäuren, ihren Salzen oder den Sulfonamid-Derivaten der erwähnten oder ähnlicher Chinolinvorstufen nach literaturbekannten Methoden herstellen. Das heißt, in den bekannten Syntheseverfahren können die erfindungsgemäßen Verbindungen überraschenderweise problemlos für die Darstellung der Farbstoffe eingesetzt werden.

### 2. Nachträgliche Derivatisierung der Farbstoffe

Erfindungsgemäß können Farbstoffe nachträglich an der 4-Methylgruppe sulfoniert werden. Ein ähnliches Verfahren ist in der Patentanmeldung WO 99/15517 für Xanthenfarbstoffe beschrieben. Überraschenderweise gelingt eine solche nachträgliche Sulfonierung auch bei vielen anderen polycyclischen Farbstoffen. Dadurch vereinfacht sich die Synthese der erfindungsgemäßen Sulfonamidderivate beträchtlich.

Die so erhaltenen sulfonierten Farbstoffe sind erstaunlicherweise ebenfalls - wie oben im Falle der Edukte beschrieben - in Sulfochloride überführbar, ohne dass der Chromophor dabei verändert oder zerstört wird. Hier wird neben Phosphoroxychlorid und/oder Phosphorpentachlorid bevorzugt eine Mischung aus Thionylchlorid und Dimethylformamid verwendet. Die Sulfochloride können als Perchlorate isoliert und gereinigt werden. Die isolierten Farbstoffsulfochloride lassen sich in bekannter Weise direkt als Marker für Aminogruppen in Biomolekülen einsetzen. Die so gewonnenen Sulfochloride können aber auch - wie oben für die Chinolin-Vorstufen beschrieben - mit fast beliebigen primären und sekundären Aminen umgesetzt werden. Man kann auf diese Weise besonders einfach die erfindungsgemäßen Farbstoffe erhalten.

Durch die Einführung der Sulfonsäuregruppe und ihre Derivatisierung werden die spektralen Eigenschaften, wie Absorptions- und Fluoreszenzmaximum, Extinktionskoeffizient sowie die Fluoreszenzquantenausbeute der Farbstoffe, praktisch nicht beeinflusst.

Durch das beschriebene Verfahren kann man aber auf einfache Weise oder sogar nachträglich den Farbstoffen - je nach verwendetem primären oder sekundären Amin - eine Vielfalt neuer Eigenschaften vermitteln. Denn das Amin kann fast beliebige weitere funktionelle Gruppen tragen. So lassen sich z.B. Cyano-, Mercapto-, Halogen-, Sulfonsäure-, Hydroxy-, Alkenyl-Gruppen etc. durch unser Verfahren in den Farbstoff einführen.

Trägt das verwendete Amin z.B. längere Alkylketten, z.B. C₁₀ bis C₃₀, so erhöht sich der lipophile Charakter, und die Verbindung ist in unpolaren Medien und Membranen löslich und kann so zum Nachweis von Membraneigenschaften oder zu molekularen Abstandsmessungen eingesetzt werden.

Die Wasserlöslichkeit eines Farbstoffs lässt sich verbessern, wenn das Amin wiederum Sulfon- oder Phosphonsäuregruppen trägt oder über Polyetherketten verfügt. Letztere machen die Verbindung auch in vielen organischen Lösungsmitteln besser löslich. Auch lassen sich dadurch ungewünschte unspezifische Wechselwirkungen mit Substrat oder Gefäßwänden vermindern. Eine Art cyclischer Polyether stellen die für den fluoreszenzsensitiven Kationennachweis verwendeten Kronenether dar, die als Aza-Derivate auch über die hier beschriebene Methode an Farbstoffmoleküle gekoppelt werden können. Ferner lassen sich nach dem beschriebenen Verfahren auch bichromöphore Systeme darstellen, in denen Energieübertragung (FRET) zwischen den Chromophoren stattfindet.

Trägt das Amin Substituenten, die zur kovalenten Kopplung fähig sind, z.B. -COOH, -NH₂, -OH oder/und -SH, so kann die erfindungsgemäß hergestellte Verbindung nach bekannten Methoden an einen Träger und/oder an ein Biomolekül gekoppelt werden. Durch dieses Vorgehen können somit Farbstoffe zur Markierung verwendet werden, die an sich nicht über eine kopplungsfähige Carboxylgruppe o.ä. verfügen, wie es bei den in WO 99/15517 beschriebenen Sulfonsäurederivaten noch erforderlich ist. Als Träger kann jedes geeignete Material ausgewählt werden, z.B. poröses Glas, Kunststoffe, lonenaustauscherharze, Dextrane, Cellulose, Cellulosederivate oder/und hydrophile Polymere. Die Biomoleküle werden vorzugsweise ausgewählt aus Peptiden, Polypeptiden, Nukleotiden, Nukleosiden, Nukleinsäuren, Nukleinsäureanaloga oder/und Haptenen.

Eine gängige Methode zur kovalenten Markierung von Biomolekülen ist die dem Fachmann vertraute Aktivestermethode. In einer bevorzugten Weise wird deshalb als Amin eine Carbonsäure mit terminaler Aminogruppe und mittlerer Kettenlänge eingesetzt. So lassen sich z.B. die käufliche 4-Methylaminobuttersäure, 4-Piperidincarbonsäure, 6-Aminohexansäure usw. als Amin in erfindungsgemäßer Weise einsetzen. Man erhält nach der Sulfonamidverknüpfung also Farbstoffe, die über eine koppelbare Carboxylgruppe verfügen. Nach der Ankopplung an primäre Aminogruppen geeigneter Biomoleküle lässt sich die verwendete Markierungsgruppe und damit der Analyt leicht durch ihre Absorption und/oder Fluoreszenz nachweisen.

Die erhaltenen Verbindungen können als Markierungsgruppen und damit der Analyt in Verfahren zur qualitativen oder/und quantitativen Bestimmung eines Analyten verwendet werden. Die Bestimmung kann in wässrigen Flüssigkeiten, z.B. Proben von Körperflüssigkeiten, wie etwa Blut, Serum, Plasma oder Urin, Abwasserproben oder Lebensmitteln, durchgeführt werden. Das Verfahren kann sowohl als Nasstest, z.B. in einer Küvette, oder als Trockentest auf einem entsprechenden Reagenzträger durchgeführt werden. Die Bestimmung der Analyten kann dabei über eine einzige Reaktion oder durch eine Sequenz von Reaktionen erfolgen. Die Verwendung der erhaltenen Verbindungen zeigt dabei sehr gute Ergebnisse in chemischen und insbesondere in medizinischen und biologischen Nachweisverfahren zur Bestimmung eines Analyten.

Die Verbindungen können in allen bekannten chemischen, medizinischen und biologischen Nachweisverfahren, in denen Fluoreszenzfarbstoffe als Markierungsgruppen geeignet sind, verwendet werden. Derartige Verfahren sind dem Fachmann bekannt und müssen deshalb nicht weiter ausgeführt werden.

In einer besonders bevorzugten Ausführungsform wird die erhaltene Verbindung kovalent an einen für den nachzuweisenden Analyten spezifischen Rezeptor gekoppelt. Der spezifische Rezeptor ist jede geeignete Verbindung oder jedes geeignete Molekül, vorzugsweise ist es ein Peptid, Polypeptid oder eine Nukleinsäure. Die Verbindungen oder Konjugate dieser Verbindungen können beispielsweise in Nukleinsäure-Hybridisierungsverfahren oder immunchemischen Verfahren verwendet werden. Derartige Verfahren sind beispielsweise beschrieben in Sambrook et al., Molecular Cloning, A Laboratory Manual, 1989, Cold Spring Harbor.

Ein wesentlicher Vorteil des beschriebenen Verfahrens ist die uneingeschränkte Anwendbarkeit auf alle Farbstoffe, für deren Synthese die oben beschriebenen Di- und Tetrahydrochinoline verwendet werden können. Man ist also in der Lage, durch die Auswahl des Chromophors genau die Farbstoffe auszuwählen, die aufgrund ihrer spektroskopischen Eigenschaften, der Lage der Absorptions- und Fluoreszenzmaxima, der Löslichkeitseigenschaften, der Fluoreszenzabklingzeit und der Höhe der Quantenausbeute für die gewünschte Anwendung besonders geeignet erscheinen.

Die Herstellung von erfindungsgemäßen Verbindungen gelingt nach den vorne beschriebenen Methoden in einfacher und kostengünstiger Weise, wie in den nachfolgenden Beispielen erläutert wird. Die so hergestellten Verbindungen sind unproblematisch handhabbar und zeigen eine gute Stabilität und Lagerfähigkeit.

Hierin beschrieben werden die Herstellung und Verwendung von Dihydrochinolin- und Tetrahydrochinolin-Derivaten entsprechend Formeln Ia und Ib mit X = OH (Sulfonsäuren); X = Cl, Br, I (Sulfonylhalogenide, bevorzugt Chloride); X = NR₁R₂ mit R₁ und R₂=substituiertes Alkyl, Aryl etc. (Sulfonamide); X = OR oder SR mit R = Alkyl, Aryl etc. (Sulfonsäureester, Thiosulfonsäure-S-Ester), sowie die Verwendung von Verbindungen entsprechend Formeln Ia und Ib zur Herstellung polycyclischer Farbstoffe entsprechend Formeln II - VII.

Hierin beschrieben werden weiterhin die Herstellung und Verwendung polycyclischer Farbstoffe entsprechend Formeln II - VII durch direkte Einführung von ein oder mehreren Substituenten SO₂X in bekannte Farbstoffe mit Dihydrochinolin- oder Tetrahydrochinolin-Endgruppen, ausgenommen X = OH in Verbindungen entsprechend Formel III mit Y = O und Formel IV.

Die Farbstoffe entsprechend Formeln II - VII werden vorzugsweise zur Markierung von Analyten, insbesondere Biomolekülen (Peptide, Nucleotide etc.), über deren NH₂- bzw. SH-Gruppen eingesetzt.

Die Farbstoffe entsprechend Formeln II - VII mit X = Cl (Sulfonylchlorid) können auch zur Kopplung mit Aminogruppen eines Analyten verwendet werden.

Ferner beschrieben wird die Verwendung der Farbstoffe entsprechend Formeln II - VII mit X = NR₁R₂ und R₁ = COOH-substituiertes Alkyl oder Aryl zur Bildung von Aktivestern (mit z.B. N-Hydroxysuccinimid) und nachfolgende Kopplung an Aminogruppen eines Analyten. Zudem können die Farbstoffe entsprechend Formeln II - VII zur Kopplung an freie Aminogruppen anderer Farbstoffe (FRET-Pärchen) verwendet werden.

Konkrete Beispiele für erfindungsgemäße Verbindungen sind in den Tabellen 1 bis 3 dargestellt.

**Tabelle 1:**

| **Chinolylsulfonsäuren und deren Derivate** | | | |
|---|---|---|---|
| Beispiel | Struktur | Summenformel | Masse MH⁺ |
| | nicht erfindungsgemäß | | |
| **1** | 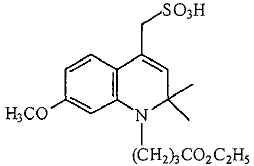 | C₁₉H₂₇NO₆S | 398 |
| | nicht erfindungsgemäß | | |
| **2** | 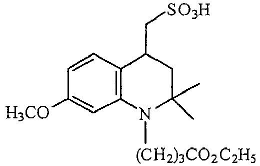 | C₁₉H₂₉NO₆S | 400 |
| | nicht erfindungsgemäß | | |
| **3** | 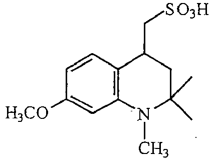 | C₁₄H₂₁NO₄S | 300 |
| | nicht erfindungsgemäß | | |
| **4** | 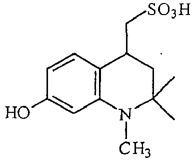 | C₁₃H₁₉NO₄S | 286 |
| | nicht erfindungsgemäß | | |
| **5** | 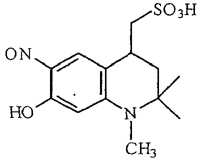 | C₁₃H₁₈N₂O₅S | 315 |
| | nicht erfindungsgemäß | | |
| **6** | 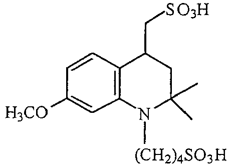 | C₁₇H₂₇NO₇S₂ | 421 |
| | nicht erfindungsgemäß | | |
| **7** | 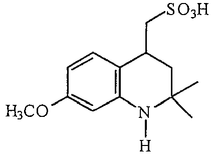 | C₁₃H₁₉NO₄S | 286 |
| | nicht erfindungsgemäß | | |
| **8** | 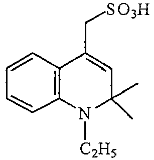 | C₁₄H₁₉N0₃S | 284 |
| | nicht erfindungsgemäß | | |
| **9** | 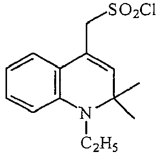 | C₁₄H₁₈ClNO₂S | 302 |
| | nicht erfindungsgemäß | | |
| **10** | 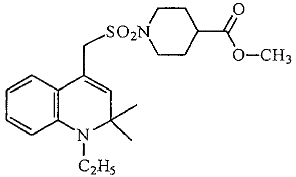 | C₂₁H₃₀N₂O₄S | 407 |
| | nicht erfindungsgemäß | | |
| **11** | 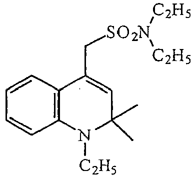 | C₁₈H₂₈N₂O₂S | 337 |
| | nicht erfindungsgemäß | | |
| **12** | 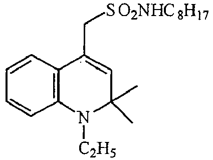 | C₂₂H₃₆N₂O₂S | 393 |
| | nicht erfindungsgemäß | | |
| **13** | 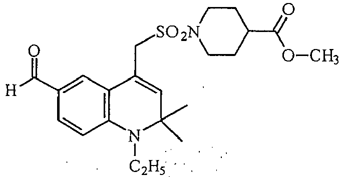 | C₂₂H₃₀N₂O₅S | 435 |
| | nicht erfindungsgemäß | | |
| **14** | 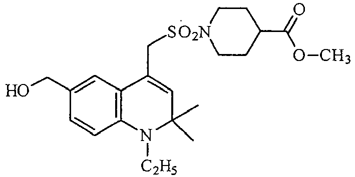 | C₂₂H₃₂N₂O₅S | 437 |
| | nicht erfindungsgemäß | | |
| **15** | 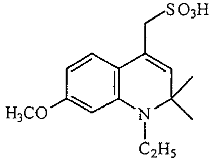 | C₁₅H₂₁NO₄S | 312 |
| | nicht erfindungsgemäß | | |
| **16** | 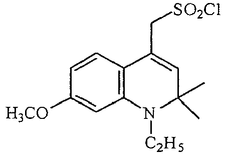 | C₁₅H₂₂ClNO₃S | 330 |
| | nicht erfindungsgemäß | | |
| **17** | 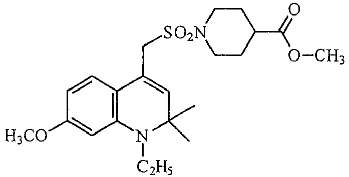 | C₂₂H₃₂N₂O₅S | 437 |
| | nicht erfindungsgemäß | | |
| **18** | 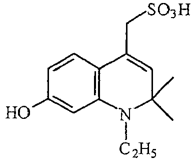 | C₁₄H₁₉NO₄S | 298 |
| | nicht erfindungsgemäß | | |
| **19** | 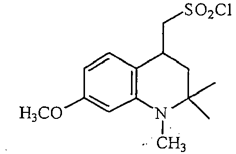 | C₁₄H₂₀ClNO₃S | 318 |
| | nicht erfindungsgemäß | | |
| **20** | 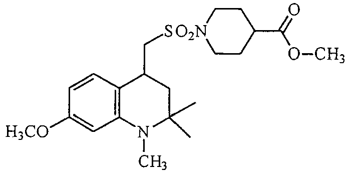 | C₂₁H₃₂N₂O₅S | 425 |
| | nicht erfindungsgemäß | | |
| **21** | 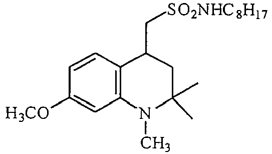 | C₂₂H₃₈N₂O₃S | 411 |
| | nicht erfindungsgemäß | | |
| **22** | 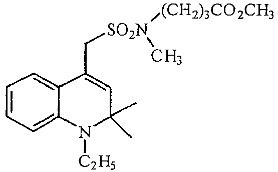 | C₂₀H₃₀N₂O₄S | 395 |
| | nicht erfindungsgemäß | | |
| **23** | 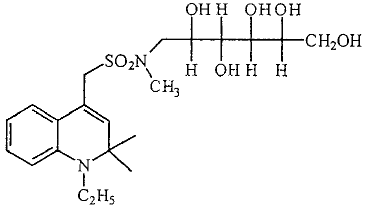 | C₂₁H₃₄N₂O₄S | 459 |
| | nicht erfindungsgemäß | | |
| **24** | 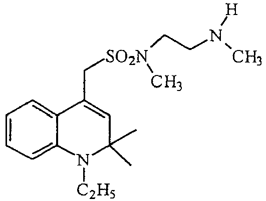 | C₁₈H₂₉N₃O₂S | 352 |
| | nicht erfindungsgemäß | | |
| **25** | 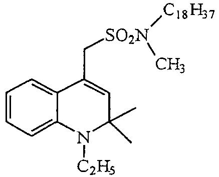 | C₃₃H₅₈N₂O₂S | 547 |
| | nicht erfindungsgemäß | | |
| **26** | 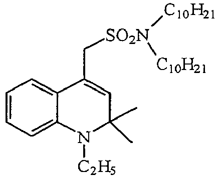 | C₃₄H₆₀N₂O₂S | 561 |
| | nicht erfindungsgemäß | | |
| **27** | 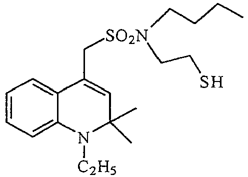 | C₂₀H₃₂N₂O₂S₂ | 397 |
| | nicht erfindungsgemäß | | |
| **28** | 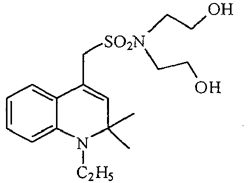 | C₁₈H₂₈N₂O₄S | 369 |
| | nicht erfindungsgemäß | | |
| **29** | 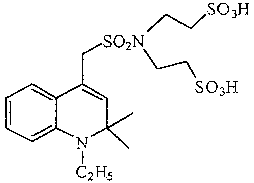 | C₁₈H₂₈N₂O₈S₃ | 497 |
| | nicht erfindungsgemäß | | |
| **30** | 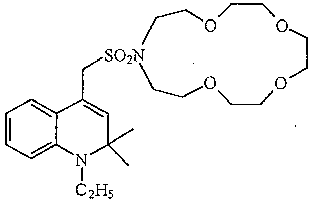 | C₂₄H₃₈N₂O₆S | 483 |
| | nicht erfindungsgemäß | | |
| **31** | 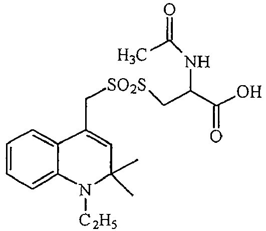 | C₁₉H₂₆N₂O₅S₂ | 427 |
| | nicht erfindungsgemäß | | |
| **32** | 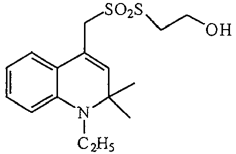 | C₁₆H₂₃NO₂S₂ | 341 |
| | nicht erfindungsgemäß | | |
| **33** | 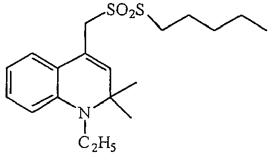 | C₁₉H₂₉NO₂S₂ | 367 |

**Tabelle 2a:**

| | | | | |
|---|---|---|---|---|
| **Cumarinfarbstoffe mit Sulfonsäure-Gruppe** | | | | |
| Spektrale Daten in Ethanol: | | λₐ Absorptionsmaximum, | | |
| | | λ_{f} Fluoreszenzmaximum | | |

| Name | Struktur | | λₐ/nm | λ_{f}/nm |
|---|---|---|---|---|
| | nicht erfindungsgemäß | | | |
| **34** | 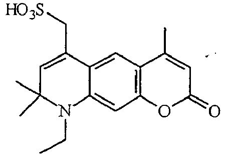 | | 394 | 468 |
| AZ 59 | | | | |
| **35** | 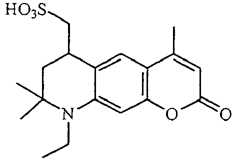 | | 383 | 455 |
| AZ 100 | | | | |
| | nicht erfindungsgemäß | | | |
| **36** | 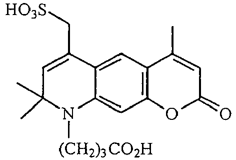 | | 394 | 469 |
| AZ 101 | | | | |

**Tabelle 2b:**

| | | | | |
|---|---|---|---|---|
| **Rhodaminfarbstoffe mit Sulfonsäure-Gruppe** | | | | |
| Spektrale Daten in Ethanol: | | λₐ Absorptionsmaximum, | | |
| | | λ_{f} Fluoreszenzmaximum | | |

| Name | Struktur (Anion A⁻) | | λₐ/nm | λ_{f}/nm |
|---|---|---|---|---|
| | nicht erfindungsgemäß | | | |
| **37** | 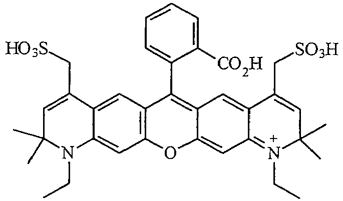 | | 592 | 612 |
| JA 317 | | | | |
| | nicht erfindungsgemäß | | | |
| **38** | 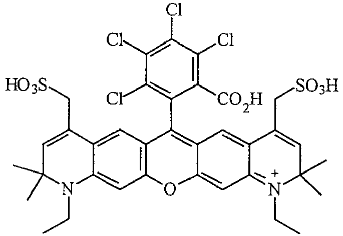 | | 621 | 642 |
| JA 325 | | | | |
| | nicht erfindungsgemäß | | | |
| **39** | 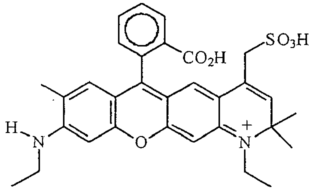 | | 562 | 587 |
| AZ 58 | | | | |
| | nicht erfindungsgemäß | | | |
| **40** | 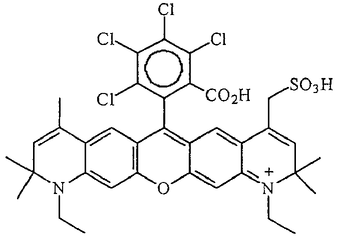 | | 623 | 645 |
| JA 407 | | | | |
| | nicht erfindungsgemäß | | | |
| **41** | 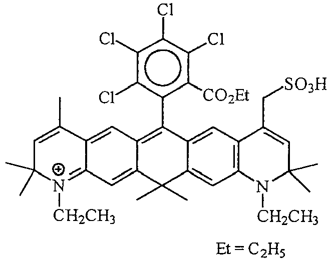 | | 623 | 644 |
| JA 407-E | | | | |

**Tabelle 2c:**

| | | | | |
|---|---|---|---|---|
| **Oxazinfarbstoffe mit Sulfonsäure-Gruppe** | | | | |
| Spektrale Daten in Ethanol: | | λₐ Absorptionsmaximum, | | |
| | | λ_{f} Fluoreszenzmaximum | | |

| Name | Struktur (Anion A⁻) | | λₐ/nm | λ_{f}/nm |
|---|---|---|---|---|
| **42** | 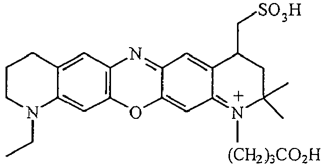 | | 655 | 680 |
| JA 378 | | | | |
| | nicht erfindungsgemäß | | | |
| **43** | 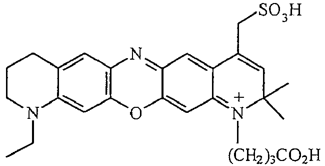 | | 675 | 699 |
| JA 379 | | | | |
| | nicht erfindungsgemäß | | | |
| **44** | 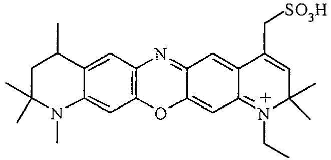 | | 674 | 699 |
| JA 322 | | | | |
| | nicht erfindungsgemäß | | | |
| **45** | 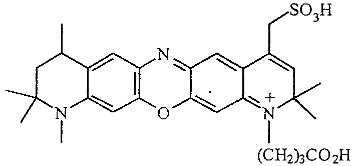 | | 673 | 698 |
| JA 324-S | | | | |
| **46** | 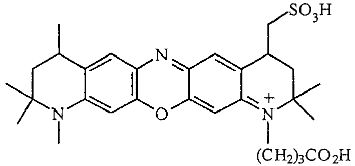 | | 655 | 679 |
| JA 326 | | | | |
| **47** | 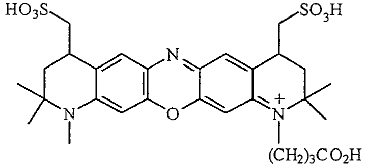 | | 654 | 678 |
| JA 329 | | | | |
| | nicht erfindungsgemäß | | | |
| **48** | 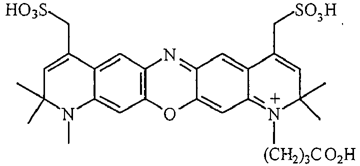 | | 694 | 717 |
| JA 410 | | | | |
| | nicht erfindungsgemäß | | | |
| **49** | 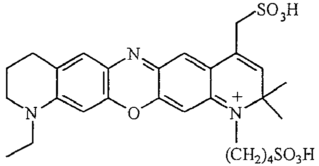 | | 674 | 695 |
| JA 331 | | | | |
| **50** | 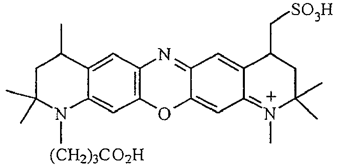 | | 654 | 678 |
| JA 366 | | | | |
| **51** | 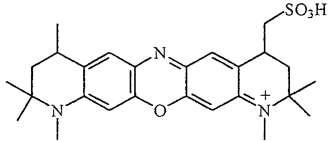 | | 653 | 676 |
| JA 403 | | | | |
| **52** | 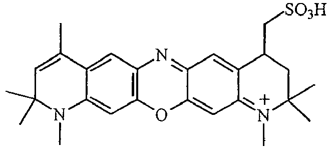 | | 678 | 699 |
| JA 404 | | | | |
| | nicht erfindungsgemäß | | | |
| **53** | 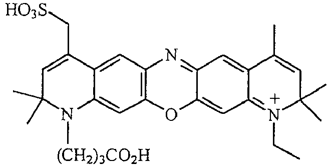 | | 694 | 715 |
| JA 408 | | | | |

**Tabelle 2d:**

| | | | | |
|---|---|---|---|---|
| **Carbopyroninfarbstoffe mit Sulfonsäure-Gruppe** | | | | |
| Spektrale Daten in Ethanol: | | λₐ Absorptionsmaximum, | | |
| | | λ_{f} Fluoreszenzmaximum | | |

| Name | Farbstoff (Anion A⁻) | | λₐ/nm | λ_{f}/nm |
|---|---|---|---|---|
| | nicht erfindungsgemäß | | | |
| **54** | 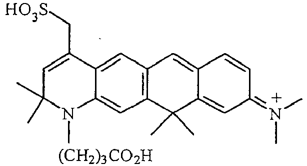 | | 637 | 664 |
| JA 323 | | | | |
| | nicht erfindungsgemäß | | | |
| **55** | 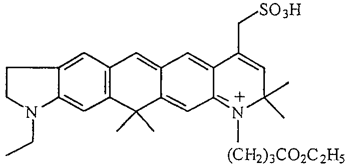 | | 648 | 675 |
| AZ 30 | | | | |
| | nicht erfindungsgemäß | | | |
| **56** | 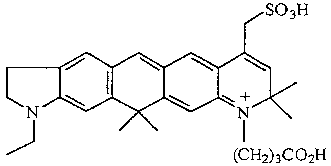 | | 648 | 674 |
| AZ 31 | | | | |
| | nicht erfindungsgemäß | | | |
| **57** | 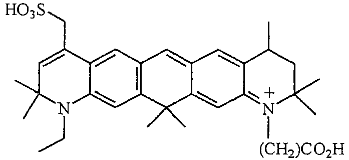 | | 649 | 674 |
| AZ 35 | | | | |
| | nicht erfindungsgemäß | | | |
| **58** | 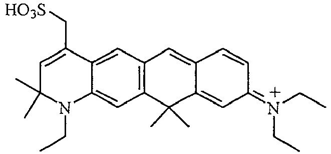 | | 641 | 666 |
| AZ 8-SO₃H | | | | |
| | nicht erfindungsgemäß | | | |
| **59** | 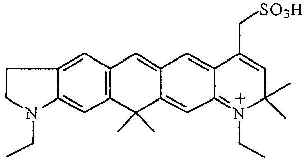 | | 647 | 675 |
| AZ 9-SO₃H | | | | |
| | nicht erfindungsgemäß | | | |
| **60** | 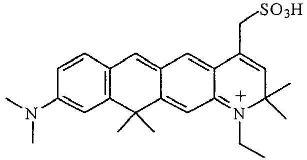 | | 637 | 664 |
| AZ 10-SO₃H | | | | |
| | nicht erfindungsgemäß | | | |
| **61** | 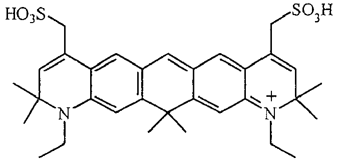 | | 664 | 688 |
| AZ 11-SO₃H | | | | |
| | nicht erfindungsgemäß | | | |
| **62** | 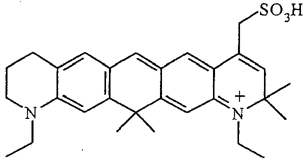 | | 647 | 674 |
| AZ 12-SO₃H | | | | |

**Tabelle 2e:**

| | | | | |
|---|---|---|---|---|
| **Amidopyryliumfarbstoffe mit Sulfonsäure-Gruppe** | | | | |
| Spektrale Daten in Ethanol: | | λₐ Absorptionsmaximum, | | |
| | | λ_{f} Fluoreszenzmaximum | | |

| Name | Struktur (Anion A⁻) | | λₐ/nm | λ_{f}/nm |
|---|---|---|---|---|
| | nicht erfindungsgemäß | | | |
| **63** | 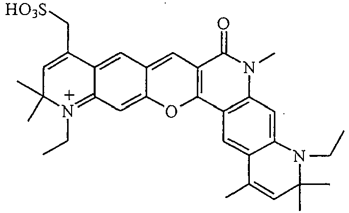 | | 635 | 695 |
| NK 32 | | | | |
| **64** | 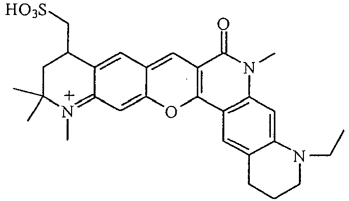 | | 610 | 668 |
| NK 34 | | | | |

**Tabelle 3a:**

| **Sulfonsäurederivate erfindungsgemäßer Cumarinfarbstoffe** | | | | |
|---|---|---|---|---|
| Spektrale Daten in Ethanol: | | λₐ Absorptionsmaximum, | | |
| | | λ_{f} Fluoreszenzmaximum | | |
| | | | | |
| Name | Struktur | | λₐ/nm | λ_{f}/nm |
| | nicht erfindungsgemäß | | | |
| **65** | 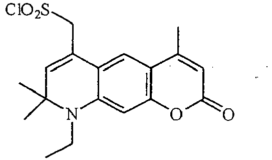 | | 394 | 468 |
| AZ 96 | | | | |
| | nicht erfindungsgemäß | | | |
| **66** | 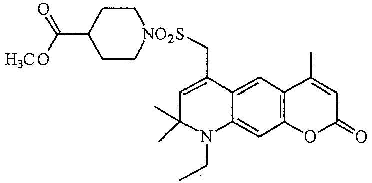 | | 393 | 468 |
| AZ 97 | | | | |
| | nicht erfindungsgemäß | | | |
| **67** | 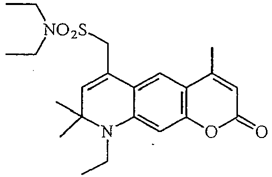 | | 394 | 467 |
| AZ 98 | | | | |
| | nicht erfindungsgemäß | | | |
| **68** | 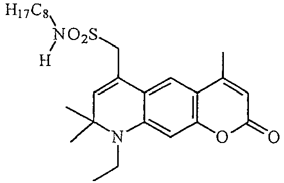 | | 393 | 469 |
| AZ 99 | | | | |

**Tabelle 3b:**

| **Sulfonsäurederivate erfindungsgemäßer Rhodaminfarbstoffe** | | | | |
|---|---|---|---|---|
| Spektrale Daten in Ethanol: | | λₐ Absorptionsmaximum, | | |
| | | λ_{f} Fluoreszenzmaximum | | |
| Name | Struktur (Anion A⁻) | | λₐ/nm | λ_{f}/nm |
| | nicht erfindungsgemäß | | | |
| **69** | 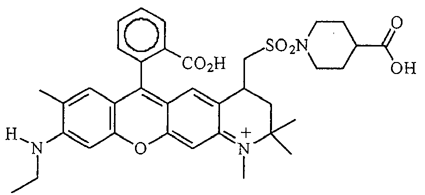 | | 542 | 567 |
| AZ 49 | | | | |
| | nicht erfindungsgemäß | | | |
| **70** | 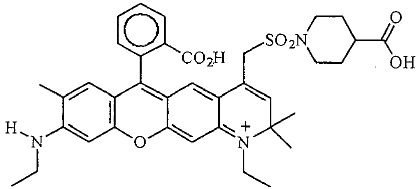 | | 561 | 587 |
| AZ 50 | | | | |
| | nicht erfindungsgemäß | | | |
| **71** | 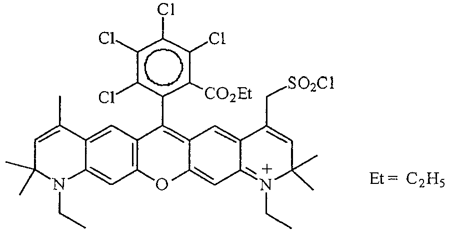 | | 624 | 644 |
| AZ 84 | | | | |
| | nicht erfindungsgemäß | | | |
| **72** | 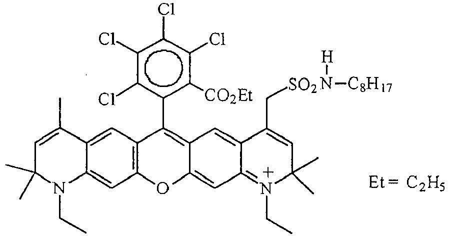 | | 624 | 644 |
| AZ 85 | | | | |
| | nicht erfindungsgemäß | | | |
| **73** | 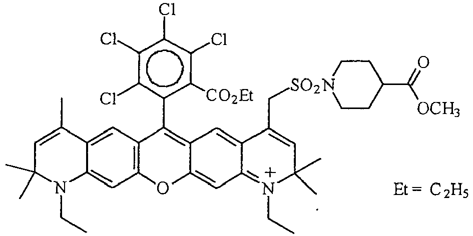 | | 624 | 645 |
| AZ 86 | | | | |
| | nicht erfindungsgemäß | | | |
| **74** | 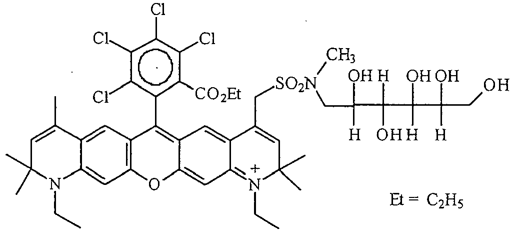 | | 623 | 644 |
| AZ 88 | | | | |
| | nicht erfindungsgemäß | | | |
| **75** | 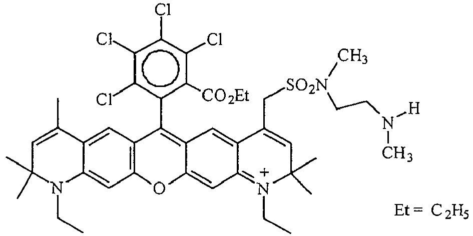 | | 624 | 644 |
| AZ 89 | | | | |
| | nicht erfindungsgemäß | | | |
| **76** | 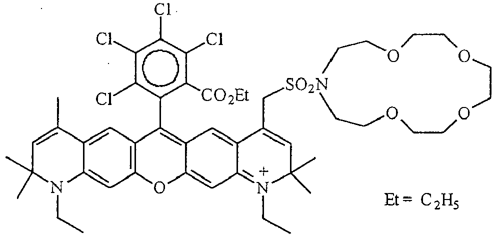 | | 624 | 645 |
| AZ 90 | | | | |
| | nicht erfindungsgemäß | | | |
| **77** | 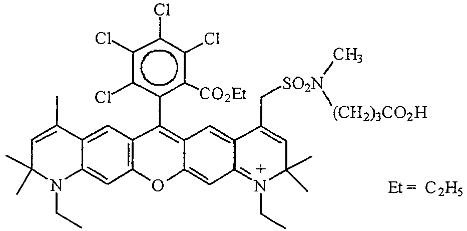 | | 624 | 644 |
| AZ 87 | | | | |
| | nicht erfindungsgemäß | | | |
| **78** | 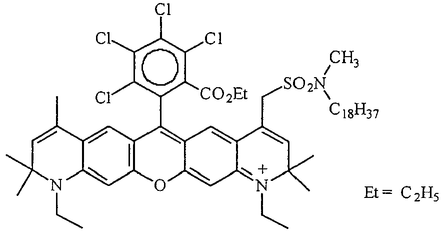 | | 624 | 644 |
| AZ 93 | | | | |
| | nicht erfindungsgemäß | | | |
| **79** | 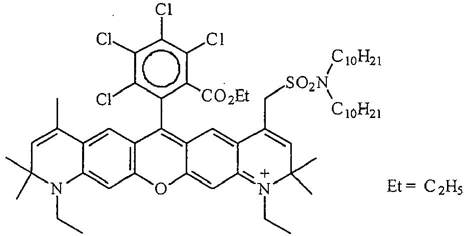 | | 624 | 646 |
| AZ 94 | | | | |
| | nicht erfindungsgemäß | | | |
| **80** | 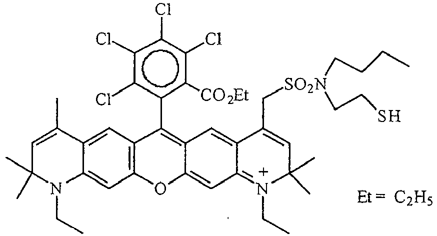 | | 624 | 644 |
| AZ 95 | | | | |
| | nicht erfindungsgemäß | | | |
| **81** | 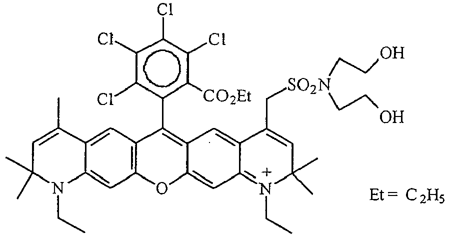 | | 624 | 646 |
| AZ 91 | | | | |
| | nicht erfindungsgemäß | | | |
| **82** | 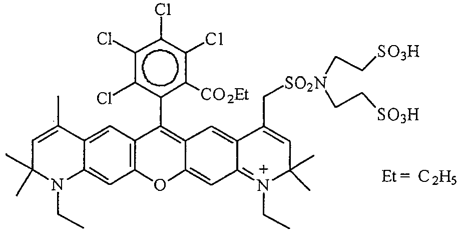 | | 624 | 644 |
| AZ 92 | | | | |

**Tabelle 3c:**

| | | | | |
|---|---|---|---|---|
| **Sulfonsäurederivate erfindungsgemäßer Oxazinfarbstoffe** | | | | |
| Spektrale Daten in Ethanol: | | λₐ Absorptionsmaximum, | | |
| | | λ_{f} Fluoreszenzmaximum | | |

| Name | Struktur (Anion A⁻) | | λₐ/nm | λ_{f}/nm |
|---|---|---|---|---|
| **83** | 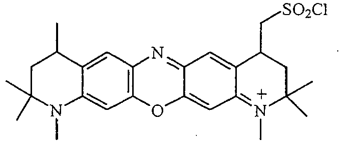 | | 654 | 680 |
| AZ 46 | | | | |
| **84** | 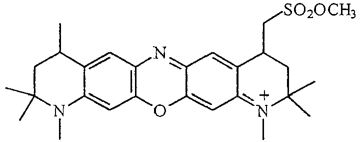 | | 654 | 679 |
| JA 403 ME | | | | |
| **85** | 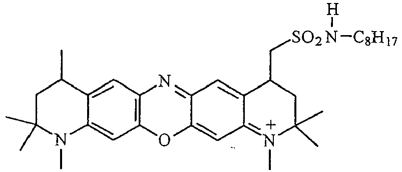 | | 653 | 677 |
| AZ 54 | | | | |
| **86** | 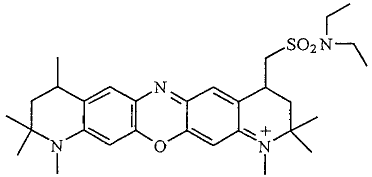 | | 654 | 679 |
| AZ 55 | | | | |
| **87** | 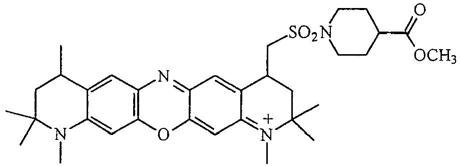 | | 653 | 678 |
| AZ 56 | | | | |
| | nicht erfindungsgemäß | | | |
| **88** | 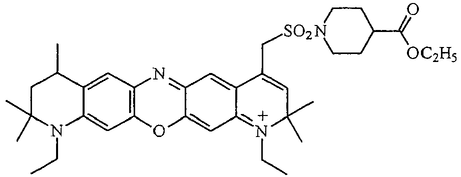 | | 678 | 699 |
| AZ 52 | | | | |
| **89** | 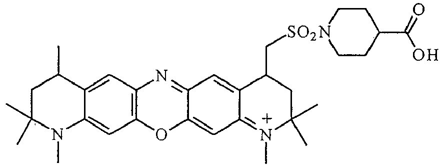 | | 653 | 677 |
| AZ 57 | | | | |
| **90** | 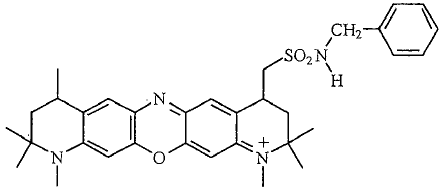 | | 654 | 678 |
| AZ 102 | | | | |
| | nicht erfindungsgemäß | | | |
| **91** | 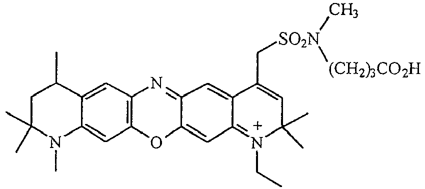 | | 678 | 717 |
| AZ 73 | | | | |
| | nicht erfindungsgemäß | | | |
| **92** | 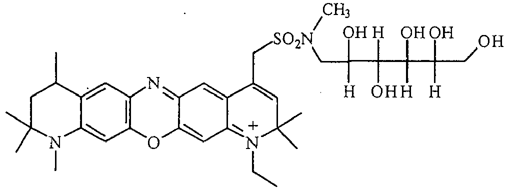 | | 678 | 715 |
| AZ 74 | | | | |
| | nicht erfindungsgemäß | | | |
| **93** | 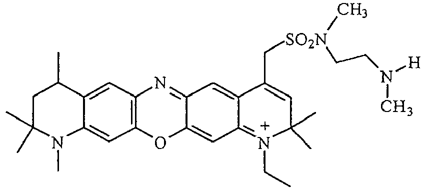 | | 679 | 715 |
| AZ 75 | | | | |
| | nicht erfindungsgemäß | | | |
| **94** | 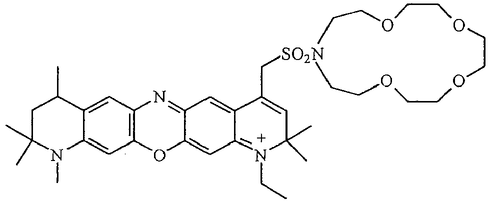 | | 678 | 715 |
| AZ 76 | | | | |
| | nicht erfindungsgemäß | | | |
| **95** | 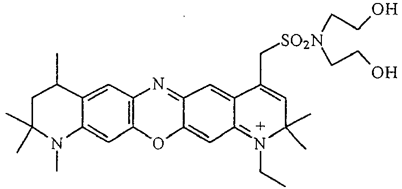 | | 678 | 716 |
| AZ 77 | | | | |
| | nicht erfindungsgemäß | | | |
| **96** | 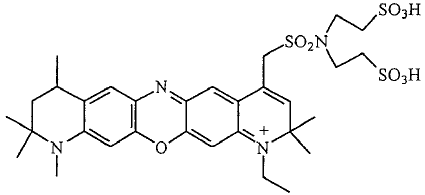 | | 677 | 714 |
| AZ 78 | | | | |
| | nicht erfindungsgemäß | | | |
| **97** | 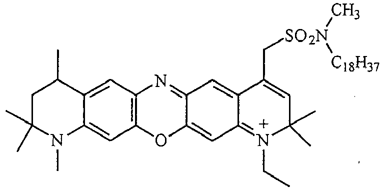 | | 678 | 716 |
| AZ 79 | | | | |
| | nicht erfindungsgemäß | | | |
| **98** | 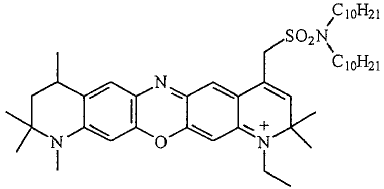 | | 678 | 715 |
| AZ 80 | | | | |
| | nicht erfindungsgemäß | | | |
| **99** | 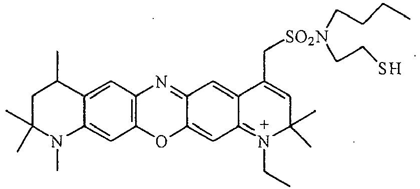 | | 679 | 715 |
| AZ 81 | | | | |

**Tabelle 3d:**

| **Sulfonsäurederivate erfindungsgemäßer Carbopyroninfarbstoffe** | | | | |
|---|---|---|---|---|
| Spektrale Daten in Ethanol: | | λₐ Absorptionsmaximum, | | |
| | | λ_{f} Fluoreszenzmaximum | | |
| | | | | |

| Name | Struktur (Anion A⁻) | | λₐ/nm | λ_{f}/nm |
|---|---|---|---|---|
| | nicht erfindungsgemäß | | | |
| **100** | 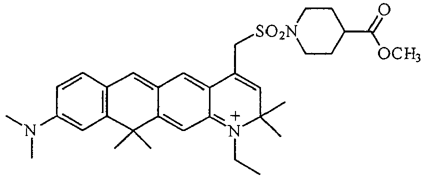 | | 634 | 658 |
| AZ 48 | | | | |
| | nicht erfindungsgemäß | | | |
| **101** | 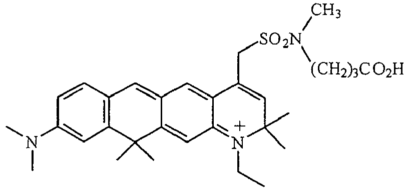 | | 635 | 661 |
| AZ 64 | | | | |
| | nicht erfindungsgemäß | | | |
| **102** | 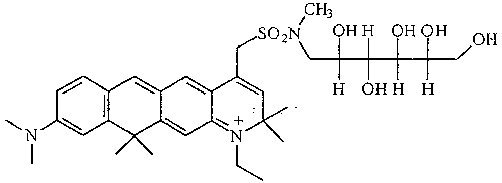 | | 634 | 661 |
| AZ 65 | | | | |
| | nicht erfindungsgemäß | | | |
| **103** | 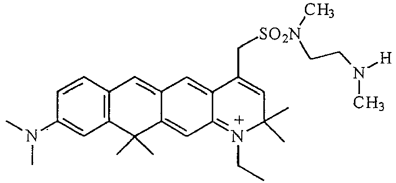 | | 635 | 663 |
| AZ 66 | | | | |
| | nicht erfindungsgemäß | | | |
| **104** | 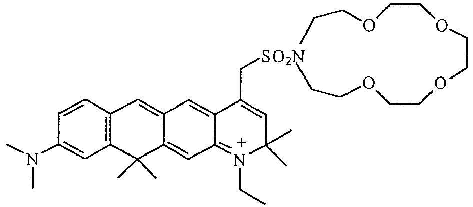 | | 635 | 663 |
| AZ 67 | | | | |
| | nicht erfindungsgemäß | | | |
| **105** | 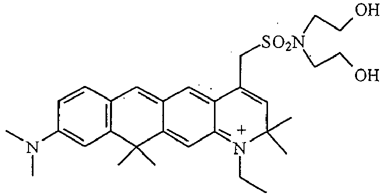 | | 634 | 662 |
| AZ 68 | | | | |
| | nicht erfindungsgemäß | | | |
| **106** | 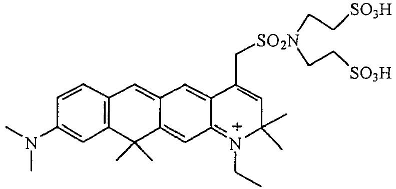 | | 634 | 661 |
| AZ 69 | | | | |
| | nicht erfindungsgemäß | | | |
| **107** | 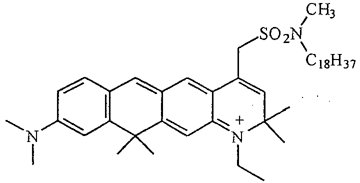 | | 634 | 662 |
| AZ 70 | | | | |
| | nicht erfindungsgemäß | | | |
| **108** | 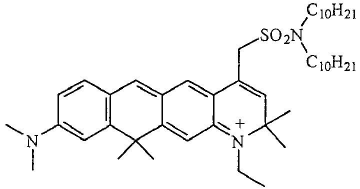 | | 634 | 662 |
| AZ 71 | | | | |
| | nicht erfindungsgemäß | | | |
| **109** | 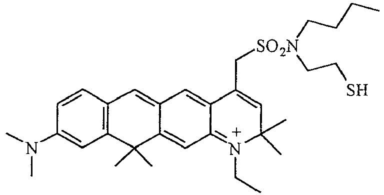 | | 635 | 661 |
| AZ 72 | | | | |
| | nicht erfindungsgemäß | | | |
| **110** | 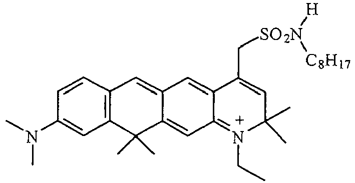 | | 634 | 661 |
| AZ 82 | | | | |
| | nicht erfindungsgemäß | | | |
| **111** | 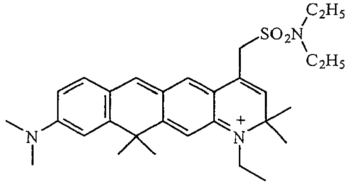 | | 635 | 663 |
| AZ 83 | | | | |

**Tabelle 3e:**

| | | | | |
|---|---|---|---|---|
| **Sulfonsäurederivate erfindungsgemäßer Trifluormethyl-substituierter Xanthenfarbstoffe** | | | | |
| Spektrale Daten in Ethanol: | | λₐ Absorptionsmaximum, | | |
| | | λ_{f} Fluoreszenzmaximum | | |
| | | | | |

| Name | Struktur (Anion A⁻) | | λₐ/nm | λ_{f}/nm |
|---|---|---|---|---|
| | nicht erfindungsgemäß | | | |
| **112** | 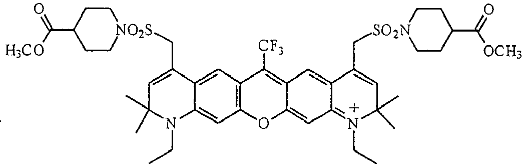 | | 665 | 694 |
| AZ 51 | | | | |
| | nicht erfindungsgemäß | | | |
| **113** | 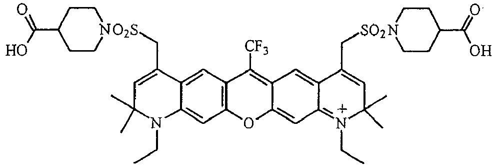 | | 664 | 694 |
| AZ 63 | | | | |
| | nicht erfindungsgemäß | | | |
| **114** | 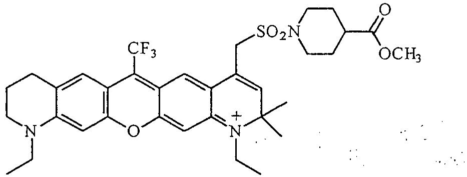 | | 651 | 680 |
| AZ 60 | | | | |
| | nicht erfindungsgemäß | | | |
| **115** | 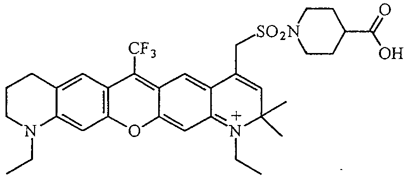 | | 652 | 680 |
| AZ 62 | | | | |
| | nicht erfindungsgemäß | | | |
| **116** | 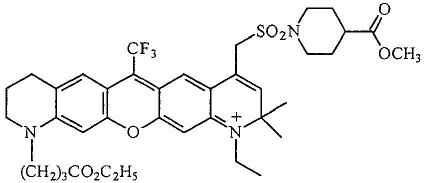 | | 652 | 679 |
| AZ 61 | | | | |

### Beispiele

Die Erfindung wird durch nachfolgende Beispiele näher erläutert. Es sind sowohl Beispiele für die Herstellung der sulfonierten Chinolinvorstufen und ihre Derivatisierung als auch Beispiele für die Synthese und Modifizierung von Farbstoffen aus den entsprechend sulfonierten Chinolinen angegeben. Die als Ausgangsverbindungen für die unter 1. beschriebenen Verbindungen verwendeten Dihydro- und Tetrahydrochinoline und primären bzw. sekundären Amine sind entweder kommerziell erhältlich oder nach literaturbekannten Synthesen oder dem Fachmann bekannten Verfahren darzustellen. Dies trifft auch für die Ausgangsfarbstoffe der unter 2. beschriebenen Farbstoffderivate zu.

### 1. Vorstufen: Herstellung nicht erfindungsgemäßer Verbindungen

### Verbindung 8 (nicht erfindungsgemäß)

Zur Darstellung von (1-Ethyl-2,2-dimethyl-1,2-dihydrochinol-4-yl)-methansulfonsäure **(8)** werden 6,0 g (21 mmol) 1-Ethyl-2,2,4-dimethyl-1,2-dihydrochinolin unter Eiskühlung in einem Gemisch aus 10 ml konzentrierter Schwefelsäure und Oleum im Verhältnis 5:1 gelöst. Es wird 20 Stunden bei Raumtemperatur verrührt. Das Reaktionsgemisch wird auf Eis gegossen und mit 20 %iger Natronlauge unter Eis/Methanol-Kühlung alkalisch gestellt (pH12). Zur Entfernung des nicht umgesetzten Edukts wird mit Chloroform extrahiert und die wässrige Phase bis zur Trockene am Rotationsverdampfer eingeengt. Der kristalline Rückstand wird in Ethanol suspendiert, vom Natriumsulfat abgesaugt und die organische Phase am Rotationsverdampfer zur Trockene eingeengt. Der harzige Rückstand kristallisiert bei Zugabe von Aceton. Es wird erneut abgesaugt, mit Aceton gewaschen und unter Vakuum über Phosphorpentoxid getrocknet. Das erhaltene Rohprodukt kann ohne weitere Reinigungsschritte in der nächsten Stufe eingesetzt werden. Ausbeute 82 %.
ESI-Massenspektrum: m/z = 282 (MH⁺)
NMR (DMSO-d₆): C**H₃**CH₂-, 0.97ppm, T, 3; -C**H₂**-N, 3.39ppm, Q, 2; 2x C**H₃**-, 1.31 ppm, S, 6; =C**H**, 5.74ppm, S, 1; -C**H₂**SO₃H, 4.27ppm, S, 2; **H** aromat, 7.15-7,58ppm, M, 4.

### Verbindung 9 (nicht erfindungsgemäß)

Zur Darstellung von (1-Ethyl-2,2-dimethyl-1,2-dihydrochinol-4-yl)-methansulfonsäure-chlorid **(9)** werden 2,5 g (8,8 mmol) (1-Ethyl-2,2-dimethyl-1,2-dihydrochinol-4-yl)-methansulfonsäure **(8)** in 70 ml trockenem Benzol suspendiert und bei Raumtemperatur portionsweise mit Phosphorpentachlorid 4,6 g (22,2 mmol) versetzt. Die Reaktionsmischung färbt sich zunächst gelb, später gelborange. Der weiße Niederschlag wird nach 1.5 Stunden abfiltriert und mit kaltem trockenem Benzol gewaschen. Es wird im Vakuum über Phosphorpentoxid getrocknet. Das erhaltene Säurechlorid wird ohne weitere Reinigung sofort in der nächsten Stufe eingesetzt.
Ausbeute 75 %.
ESI-Massenspektrum: m/z = 300 (MH⁺)

### Verbindung 10 (nicht erfindungsgemäß)

Zur Darstellung von (1-Ethyl-2,2-dimethyl-1,2-dihydrochinol-4-yl-methansulfonyl)-piperidin-4-carbonsäuremethylester **(10)** werden 2 g (6,6 mmol) (1-Ethyl-2,2-dimethyl-1,2-dihydrochinol-4-yl)-methansulfonsäurechlorid **(9)** in 40 ml trockenem Acetonitril gelöst und mit einem Eisbad gekühlt. Man versetzt tropfenweise mit 1,13 g (7,9 mmol) 4-Piperidincarbonsäuremethylester gefolgt von 0,85 g (6,6 mmol) Diisopropylethylamin. Die Reaktionsmischung wird 30 Minuten bei Raumtemperatur verrührt. Man versetzt mit Wasser und extrahiert mit Chloroform, wäscht mit 10 %iger kalter Sodalösung und trocknet über wasserfreiem Natriumsulfat. Das Lösungsmittel wird am Rotationsverdampfer abdestilliert und der Rückstand an Kieselgel mit einem von Chloroform nach Ethanol verlaufenden Gradienten säulenchromatographisch gereinigt. Ausbeute 82 %.
ESI-Massenspektrum: m/z = 407 (MH⁺)

### Verbindung 13 (nicht erfindungsgemäß)

Zur Darstellung von 1-(1-Ethyl-6-formyl-2,2-dimethyl-1,2-dihydrochinol-4-yl-methan-sulfonyl)-piperidin-4-carbonsäuremethylester **(13)** werden 3 ml trockenes Dimethylformamid im Eis/Methanol Bad auf -10 °C gekühlt und tropfenweise mit 0,45 ml (4,9 mmol) Phosphoroxidtrichlorid versetzt. Die Reaktionsmischung wird 20 Minuten bei -5 °C verrührt. (1-Ethyl-2,2-dimethyl-1,2-dihydrochinol-4-yl-methansulfonyl)-piperidin-4-carbonsäure-methylester **(10)** werden in 1,5 ml trockenem Dimethylformamid gelöst und bei -5 °C dem Reaktionsgemisch zugetropft. Nach beendeter Zugabe wird 50 Minuten auf 80 °C erhitzt. Die Reaktionslösung wird auf Eiswasser gegossen und mit Chloroform versetzt. Es wird mit 20 %iger Natronlauge auf pH12 eingestellt und mehrmals mit Chloroform extrahiert. Die organische Phase wird mit 10 %iger Sodalösung gewaschen und über wasserfreiem Natriumsulfat getrocknet. Das erhaltene Rohprodukt wird am Rotationsverdampfer unter Vakuum zur Trockene eingeengt und an Kieselgel über einem von Chloroform nach Ethanol verlaufenden Gradienten säulenchromatographisch gereinigt.
Ausbeute 88 %.
ESI-Massenspektrum: m/z = 435 (MH⁺)
NMR (CDCl₃): C**H₃**CH₂-, 1.22ppm, T, 3; -C**H₂**-N, 3.41 ppm, Q, 2; 2x C**H₃**-, 1.42ppm, S, 6; =C**H**, 5.63ppm, S, 1; -C**H₂**SO₂N, 4.00ppm, S, 2; **H**-5 aromat, 7.59ppm, S, 1; **H**-7 aromat, 7.57ppm, D, 1; **H**-8 aromat, 6.55ppm, D, 1; **H-**C=O, 9.68ppm, S, 1; -O-C**H₃**, 3.63ppm, S, 1; C**H**(Piperidin)-C=O, 2.31 ppm, M, 1; N-C**HH'**(Piperidin), 2.83ppm u. 3.52ppm; M, 2,2; HC-C**HH'**(Piperidin), 1.67ppm u. 1.83ppm; M, 2,2.

### Verbindung 14 (nicht erfindungsgemäß)

Zur Darstellung von 1-(1-Ethyl-6-hydroxymethyl-2,2-dimethyl-1,2-dihydrochinol-4-yl-methansulfonyl)-piperidin-4.-carbonsäuremethylester **(14)** werden 1 g (2,3 mmol) 1-(1-Ethyl-6-formyl-2,2-dimethyl-1,2-dihydrochinol-4-yl-methansulfonyl)-piperidin-4-carbonsäuremethylester **(13)** in 10 ml Ethanol gelöst und unter Eiskühlung mit 0,05g Natriumborhydrid versetzt, wobei eine deutliche Gasentwicklung mit Schäumen zu beobachten ist. Es wird 50 Minuten bei Raumtemperatur verrührt. Zur Zersetzung des Überschusses an Reduktionsmittel wird tropfenweise mit 1 N Salzsäure versetzt bis kein Schäumen mehr zu beobachten ist (pH7). Man gießt auf 50 ml Wasser und extrahiert 3 mal mit 20 ml Chloroform. Die vereinigten organischen Phasen werden über wasserfreiem Natriumsulfat getrocknet und das Lösungsmittel am Rotationsverdampfer bis zur Trockene abdestilliert. Der erhaltene Rückstand wird an Kieselgel mit einem von Chloroform nach Ethanol verlaufenden Gradienten säulenchromatographisch gereinigt.
Ausbeute 88 %.
ESI-Massenspektrum: m/z = 435 (MH⁺)
NMR (CDCl₃): C**H₃**CH₂-, 1.10ppm, T, 3; -C**H₂**-N, 3.25ppm, Q, 2; 2x C**H₃**-, 1.27ppm, S, 6; =C**H,** 5.53ppm, S, 1; -C**H₂**SO₂N, 3.96ppm, S, 2; **H**-5 aromaten, 7.10ppm, S, 1; **H**-7 aromaten, 7.05ppm, D, 1; **H**-8 aromaten, 6.46ppm, D, 1; **H₂**C-OH, 4.47ppm, S, 1; -O-C**H₃**, 3.57ppm, S, 1; C**H**(Piperidin)-C=O, 2.29ppm, M, 1; N-C**HH'**(Piperidin), 2.82ppm u. 3.53ppm; M, 2,2; HC-C**HH'**(Piperidin), 1.68ppm u. 1.73ppm; M, 2,2; -CH₂-O**H**, nn.

### Verbindung 3 (nicht erfindungsgemäß)

Zur Darstellung von (1-Methyl-7-methoxy-2,2-dimethyl-1,2,3,4-tetrahydrochinol-4-yl)-methansulfonsäure **(3)** werden 5,0 g (1-Methyl-7-methoxy-2,2-dimethyl-1,2-dihydrochinol-4-yl)-methansulfonsäure werden in 100 ml Methanol gelöst und mit 0,5 g 10 %igem Palladium auf Aktivkohle versetzt. Das Reaktionsgemisch wird im Autoklaven 18 Stunden bei 70 bar und Raumtemperatur hydriert. Man filtriert und engt am Rotationsverdampfer zur Trockene ein. Der erhaltene Feststoff lässt sich ohne weitere Reinigung in der nächsten Stufe einsetzen.
Ausbeute 90 %.
ESI-Massenspektrum: m/z = 270 (MH⁺)

### Verbindung 11 (nicht erfindungsgemäß)

Die Darstellung von N,N-Diethyl-(1-Ethyl-2,2-dimethyl-1,2-dihydrochinol-4-yl)-methansulfonamid **(11)** erfolgt analog Verbindung **(10)** unter Verwendung von Verbindung **(9)** mit Diethylamin. Die säulenchromatographische Isolierung des Produktes erfolgt an Kieselgel mit einem von Chloroform nach Ethanol verlaufenden Gradienten.
Ausbeute 79 %.
ESI-Massenspektrum: m/z = 337 (MH⁺)

### Verbindung 12 (nicht erfindungsgemäß)

Die Darstellung von (1-Ethyl-2,2-dimethyl-1,2-dihydrochinol-4-yl)-N-octyl-methansulfonamid **(12)** erfolgt analog Verbindung **(10)** unter Verwendung von Verbindung **(9)** mit n-Octylamin. Die säulenchromatographische Isolierung des Produktes erfolgt an Kieselgel mit einem von Chloroform nach Ethanol verlaufenden Gradienten.
Ausbeute 81 %
ESI-Massenspektrum: m/z = 393 (MH⁺)

### Verbindung 15 (nicht erfindungsgemäß)

Zur Darstellung von (1-Ethyl-7-methoxy-2,2-dimethyl-1,2-dihydrochinol-4-yl)-methansulfonsäure **(15)** werden 20 g (8,6 mmol) 1-Ethyl-7-methoxy-2,2,4-dimethyl-1,2-dihydrochinolin unter Eiskühlung in einem Gemisch aus 10 ml konzentrierter Schwefelsäure und Oleum im Verhältnis 5:1 gelöst. Es wird 20 Stunden bei Raumtemperatur verrührt. Das Reaktionsgemisch wird auf Eis gegossen und mit 20 %iger Natronlauge unter Eis/Methanol Kühlung alkalisch gestellt (pH12). Zur Entfernung des nicht umgesetzten Edukts wird mit Chloroform extrahiert und die wässrige Phase zur Trockene am Rotationsverdampfer eingeengt. Der kristalline Rückstand wird säulenchromatographisch an Kieselgel mit einem von Chloroform nach Ethanol verlaufenden Gradienten gereinigt.
Ausbeute 86 %.
ESI-Massenspektrum: m/z = 312 (MH⁺)
NMR (DMSO-d₆): C**H₃**CH₂-, 1.09ppm, T, 3; -C**H₂**-N, 3.25ppm, Q, 2; 2x C**H₃**-, 1.24ppm, S, 6; =C**H**, 5.28ppm, S, 1; -C**H₂**SO₃H, 3.43ppm, S, 2; **H**-5 aromat, 6.04ppm, D, 1; **H**-6 aromat, 7.17ppm, D, 1; **H**-8 aromat, 5.93ppm, S, 1; CH₃O-, 3.68ppm, S, 3.

### Verbindung 16 (nicht erfindungsgemäß)

Die Darstellung von (1-Ethyl-7-methoxy-2,2-dimethyl-1,2-dihydrochinol-4-yl)-methan-sulfonsäurechlorid **(16)** erfolgt analog Verbindung **(9)** aus Verbindung **(15)** und Phosphorpentachlorid in trockenem Benzol. Das erhaltene Säurechlorid wird sofort zur weiteren Umsetzung eingesetzt.
Ausbeute 73 %.
ESI-Massenspektrum: m/z = 330 (MH⁺)

### Verbindung 17 (nicht erfindungsgemäß)

Die Darstellung von (1-Ethyl-7-methoxy-2,2-dimethyl-1,2-dihydrochinol-4-yl-methansulfonyl)-piperidin-4-carbonsäuremethylester **(17)** erfolgt analog Verbindung **(10)** aus Verbindung **(16)** in trockenem Acetonitril bei Raumtemperatur.
Ausbeute 69 %.
ESI-Massenspektrum: m/z = 437 (MH⁺)
NMR (CDCl₃): C**H₃**CH₂-, 1.15ppm, T, 3; -C**H₂**-N, 3.27ppm, Q, 2; 2x C**H₃**-, 1.31ppm, S, 6; =C**H**, 5.41 ppm, S, 1; -C**H₂**SO₂N, 3.97ppm, S, 2; **H**-5 aromat, 6.17ppm, D, 1; **H**-6 aromat, 7.07ppm, D, 1; **H**-8 aromat, 6.09ppm, S, 1; -O-C**H₃**, 3.77ppm, S, 1; C**H**(Piperidin)-C=O, 2.31ppm, M, 1; N-C**HH'**(Piperidin), 2.82ppm u. 3.63ppm; M, 2,2; HC-C**HH'**(Piperidin), 1.63ppm u. 1.80ppm; M, 2,2; O=C-OC**H₃**, 3.63, S, 3.

### Verbindung 18 (nicht erfindungsgemäß)

Zur Darstellung von (1-Ethyl-7-hydroxy-2,2-dimethyl-1,2-dihydrochinol-4-yl)-methan-sulfonsäure **(18)** werden 5 g (16,1 mmol) (1-Ethyl-7-methoxy-2,2-dimethyl-1,2-dihydrochinol-4-yl)-methansulfonsäure **(16)** in 10 ml 48 %iger Bromwasserstoffsäure suspendiert und 2 Stunden zum Rückfluss erhitzt. Man gießt auf Eis, versetzt mit Chloroform und neutralisiert mit 20 %iger Natronlauge. Die organische Phase wird abgetrennt und die wässrige Phase am Rotationsverdampfer im Vakuum zur Trockene einrotiert. Der Rückstand wird in heißem Ethanol suspendiert, filtriert und das Filtrat erneut bis zur Trockene einrotiert. Das so erhaltene Produkt wird ohne weitere Reinigung in der nächsten Stufe eingesetzt.
Ausbeute 91 %.
ESI-Massenspektrum: m/z = 298 (MH⁺)

### Verbindung 19 (nicht erfindungsgemäß)

Die Darstellung von (1-Methyl-7-methoxy-2,2-dimethyl-1,2,3,4-tetrahydrochinol-4-yl)-methansulfonsäurechlorid **(19)** erfolgt analog Verbindung **(16)** unter Verwendung von Verbindung **(3)** und Phosphorpentachlorid mit dem Unterschied, dass 18 Stunden bei Raumtemperatur verrührt wird.
Ausbeute 78 %.
ESI-Massenspektrum: m/z = 317 (MH⁺)

### Verbindung 20 (nicht erfindungsgemäß)

Die Darstellung von (1-Methyl-7-methoxy-2,2-dimethyl-1,2,3,4-tetrahydrochinol-4-yl-methansulfonyl)-piperidin-4-carbonsäuremethylester **(20)** erfolgt analog Verbindung **(10)** aus Verbindung **(19)** mit 4-Piperidincarbonsäuremethylester in trockenem Acetonitril. Die säulenchromatographische Isolierung erfolgt an Kieselgel mit einem von Chloroform nach Ethanol verlaufenden Gradienten.
Ausbeute 69 %.
ESI-Massenspektrum: m/z = 425 (MH⁺)

### Verbindung 21 (nicht erfindungsgemäß)

Die Darstellung von (1-Methyl-7-methoxy-2,2-dimethyl-1,2,3,4-tetrahydrochinol-4-yl)-N-octyl-methansulfonamid **(21)** erfolgt analog Verbindung **(10)** aus Verbindung **(19)** mit n-Octylamin in trockenem Acetonitril. Die säulenchromatographische Isolierung erfolgt an Kieselgel mit einem von Chloroform nach Ethanol verlaufenden Gradienten.
Ausbeute 69 %.
ESI-Massenspektrum: m/z = 411 (MH⁺)

### 2. Farbstoffe: Herstellung erfindungsgemäßer Verbindungen

### Verbindung 55 (AZ 30) (nicht erfindungsgemäß)

1,2 g (4,93 mmol) 4-(6-Hydroxymethyl-2,2,4-trimethyl-2H-chinol-1-yl)-buttersäure-ethylester werden zusammen mit 0,72 g (4,93 mmol) 1-Ethyl-6-isopropenyl-indolin in 20 ml trockenem Dichlormethan gelöst und auf -5 °C gekühlt. Man versetzt tropfenweise mit 7 ml einer 1 molaren Lösung von Bortrichlorid in Dichlormethan. Das Reaktionsgemisch wird 2 Stunden bei Raumtemperatur verrührt, das Lösungsmittel am Rotationsverdampfer entfernt und der Rückstand in konzentrierter Schwefelsäure gelöst. Die Reaktionsmischung wird 4 Stunden bei Raumtemperatur verrührt. Man tropft in eiskaltes Ethanol, versetzt mit 0,8 g Tetrabutylammoniummetaperiodat und erhitzt kurz zum Sieden. Man lässt abkühlen, versetzt mit Wasser und nimmt den Farbstoff in Chloroform auf. Es wird über wasserfreiem Natriumsulfat getrocknet und die Farbstofflösung am Rotationsverdampfer zur Trockene eingeengt. Das erhaltene Farbstoffrohprodukt wird an Kieselgel mit einem von Chloroform nach Ethanol verlaufenden Gradienten säulenchromatographisch gereinigt.
Ausbeute 45 %.
ESI-Massenspektrum: m/z = 565 (M⁺)
Absorptionsmaximum: λₐ = 648 nm (in Ethanol)

### Verbindung 83 (AZ 46)

100 mg (0,21 mmol) JA 403 werden in 8 ml trockenem Acetonitril gelöst und im kalten Wasserbad gekühlt. Man versetzt mit 4 Tropfen trockenem Dimethylformamid gefolgt von 6 Tropfen frisch destilliertem Thionylchlorid. Es wird 45 Minuten verrührt. Die Umsetzung erfolgt quantitativ. Das Reaktionsgemisch wird in einem Eisbad gekühlt. Die Farbstofflösung wird in eine mit Eis/Methanol gekühlte 20 %ige Natriumperchloratlösung getropft. Der sich in feinen Kristallen abscheidende Farbstoff wird abgesaugt, mit wenig kaltem Wasser gewaschen und im Ölpumpenvakuum über Phosphorpentoxid getrocknet. Der Farbstoff kann ohne zusätzliche Reinigung für weitere Umsetzungen eingesetzt werden.
Ausbeute 81 %.
ESI-Massenspektrum: m/z = 502 (M⁺)
Absorptionsmaximum: λₐ = 650 nm (Acetonitril)

### Verbindung 90 (AZ 102)

10 mg (0.017 mmol) AZ 46 werden in 5 ml trockenem Acetonitril gelöst und mit 5 ml einer 10 mmolaren Lösung aus Benzylamin in trockenem Acetonitril versetzt. Das Reaktionsgemisch wird 20 Minuten bei Raumtemperatur verrührt. Die Reaktionslösung wird zur Trockene einrotiert und an Kieselgel mit einem von Chloroform nach Ethanol verlaufenden Gradienten säulenchromatographisch gereinigt. Ausbeute 88 %.
ESI-Massenspektrum: m/z = 573 (M⁺)
Absorptionsmaximum: λₐ = 653 nm (in Ethanol)

### Verbindung 87 (AZ 56)

Die Synthese erfolgt analog zur Darstellung von AZ 102 unter Verwendung einer 10 mmolaren Lösung von 4-Piperidincarbonsäuremethylester. Die Isolierung erfolgt säulenchromatographisch an Kieselgel mit einem von Chloroform nach Ethanol verlaufenden Gradienten.
Ausbeute 91 %.
ESI-Massenspektrum: m/z = 609 (M⁺)
Absorptionsmaximum: λₐ = 652 (in Ethanol)

### Verbindung 89 (AZ 57)

10 mg (0,014 mmol) AZ 56 werden in 20 ml Acetonitril Wasser 1:1 gelöst und mit 0,5 ml 2 N Salzsäure versetzt. Die Reaktionsmischung wird 7 Stunden zum Rückfluss erhitzt. Die Farbstofflösung wird unter Vakuum eingeengt und an Kieselgel mit einem von Chloroform nach Ethanol verlaufenden Gradienten säulenchromatographisch gereinigt.
Ausbeute 62 %.
ESI-Massenspektrum: m/z = 595 (M⁺)
Absorptionsmaximum: λₐ = 652 nm (in Ethanol)

### Verbindung 34 (AZ 59) (nicht erfindungsgemäß)

500 mg (1,7 mmol) (1-Ethyl-7-hydroxy-2,2-dimethyl-1,2-dihydroChinol-4-yl)-methan-sulfonsäure (18), 440 mg (3,4 mmol) Acetessigsäureethylester und 460 mg trockenes Zinkchlorid werden in 40 ml absolutem Ethanol suspendiert und 24 Stunden zum Rückfluss erhitzt. Die Reaktionsmischung wird filtriert, am Rotationsverdampfer bis zur Trockene eingeengt und der Rückstand an Kieselgel mit einem von Chloroform nach Ethanol verlaufenden Gradienten säulenchromatographisch gereinigt.
Ausbeute 60 %.
ESI-Massenspektrum: m/z = 364 (MH⁺)
Absorptionsmaximum: λₐ = 394 nm (in Ethanol)

### Verbindung 71 (AZ 84) (nicht erfindungsgemäß)

Die Synthese erfolgt analog AZ 46 unter Verwendung von JA 407-E mit Thionylchlorid/DMF in trockenem Acetonitril. Der erhaltene Farbstoff wird als Perchlorat isoliert und getrocknet. Das Farbstoffsulfochlorid kann ohne weitere Reinigung sofort weiter umgesetzt werden.
Ausbeute 81 %.
ESI-Massenspektrum: m/z = 811 (M⁺)
Absorptionsmaximum: λₐ = 623 nm (in Ethanol)

### Verbindung 72 (AZ 85) (nicht erfindungsgemäß)

Die Darstellung erfolgt analog AZ 102 unter Verwendung von AZ 84 und n-Octylamin in trockenem Acetonitril. Die säulenchromatographische Isolierung erfolgt an Kieselgel mit einem von Chloroform nach Ethanol verlaufenden Gradienten.
Ausbeute 73 %.
ESI-Massenspektrum: m/z = 904 (M⁺)

### Verbindung 65 (AZ 96) (nicht erfindungsgemäß)

Die Darstellung erfolgt analog Verbindung (9) aus AZ 59 mit Phosphorpentachlorid in Benzol wobei 6 Stunden bei Raumtemperatur verrührt wird.
Ausbeute 63 %.
ESI-Massenspektrum: m/z = 383.2 (MH⁺)
Absorptionsmaximum: λₐ = 392 nm (in Ethanol)

### Verbindung 66 (AZ 97) (nicht erfindungsgemäß)

Die Darstellung erfolgt analog AZ 102 aus AZ 96 mit 4-Piperidincarbonsäuresäureethylester. Die säulenchromatographische Isolierung erfolgt an Kieselgel mit einem von Chloroform nach Ethanol verlaufenden Gradienten.
Ausbeute 76 %.
ESI-Massenspektrum: m/z = 489.2 (MH⁺)
Absorptionsmaximum: λₐ = 393 nm (in Ethanol)

### Verbindung 84 (JA 403 ME)

100 mg (0,17 mmol) JA 403 werden mit 0,5 ml Dimethylsulfat in 20 ml trockenem Acetonitril gelöst und für ca. 5 Stunden zum Rückfluss erhitzt. Die Reaktion wird dünnschicht-chromatographisch verfolgt. Nach Beendigung der Umsetzung wird die Reaktionsmischung zur Trockne eingeengt, in wenig Chloroform aufgenommen und der Rückstand an Kieselgel mit einem von Chloroform nach Ethanol verlaufenden Gradienten säulenchromatographisch gereinigt.
Ausbeute 70 %.
ESI-Massenspektrum: m/z = 498 (M⁺)
Absorptionsmaximum: λₐ = 654 nm (in Ethanol)

Ferner werden die folgenden Punkte beschrieben:
Punkt 1:
   Verfahren zur Herstellung von Dihydrochinolinverbindungen der allgemeinen Formel Ia oder von Tetrahydrochinolinverbindungen der allgemeinen Formel Ib 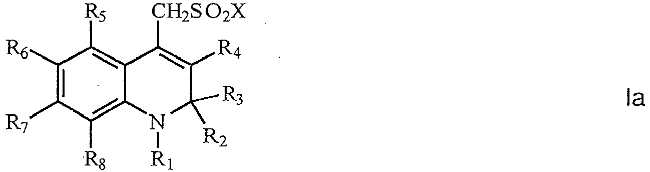 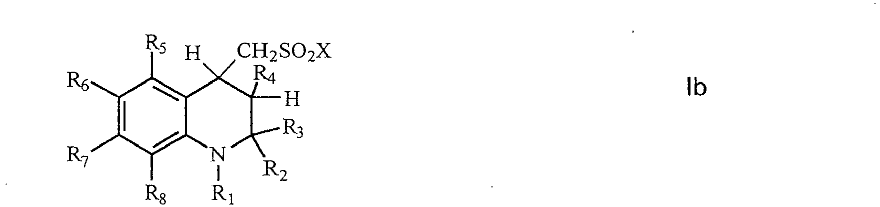 worin R₁ Wasserstoff oder eine Kohlenwasserstoffgruppe mit 1-20 C-Atomen bedeutet, wobei die Kohlenwasserstoffgruppe gegebenenfalls ein oder mehrere Heteroatome oder/und einen oder mehrere Substituenten enthalten kann,
   R₂, R₃, R₄, R₅, R₆, R₇ und R₈ bei jedem Auftreten unabhängig voneinander Wasserstoff, Halogen, eine Hydroxy-, Amino-, Sulfo-, Carboxy- oder Aldehydgruppe oder eine Kohlenwasserstoffgruppe mit 1-20 C-Atomen bedeutet, wobei die Kohlenwasserstoffgruppe gegebenenfalls ein oder mehrere Heteroatome oder/und einen oder mehrere Substituenten enthalten kann, oder die Reste R₁ und R₈ zusammen ein Ringsystem bilden und
   X OH, Halogen, -O-R₉, -S-R₁₀ oder -NR₁₁R₁₂ bedeutet, wobei R₉, R₁₀, R₁₁ und R₁₂ unabhängig voneinander Wasserstoff oder einen C1 bis C20 Kohlenwasserstoffrest bedeuten, welcher gegebenenfalls ein oder mehrere Heteroatome oder/und einen oder mehrere Substituenten enthalten kann,
   **dadurch gekennzeichnet,**
   dass man die entsprechenden Verbindungen I'a 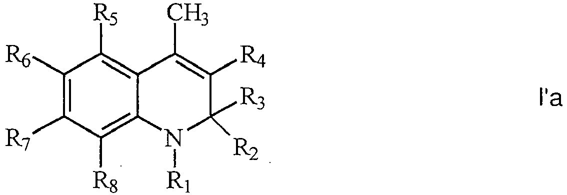 zu Ia (X = OH) sulfoniert und gegebenenfalls durch Hydrierung zu Ib (X = OH) umsetzt.
Punkt 2:
   Verfahren nach Punkt 1,
   **dadurch gekennzeichnet,**
   dass man die Sulfonierung mittels konzentrierter Schwefelsäure durchführt.
Punkt 3:
   Verfahren nach Punkt 1 oder 2,
   **dadurch gekennzeichnet,**
   dass man die bei der Sulfonierung gebildete Sulfonsäuregruppe derivatisiert.
Punkt 4:
   Verfahren nach Punkt 3,
   **dadurch gekennzeichnet,**
   dass man die Sulfonsäuregruppe zum Sulfochlorid umsetzt.
Punkt 5:
   Verfahren nach Punkt 3 oder 4,
   **dadurch gekennzeichnet,**
   dass man die Sulfochloridgruppe mit einem primären oder sekundären Amin zum Sulfonamid umsetzt.
Punkt 6:
   Dihydrochinolinverbindung der allgemeinen Formel Ia oder Tetrahydrochinolinverbindung der allgemeinen Formel Ib 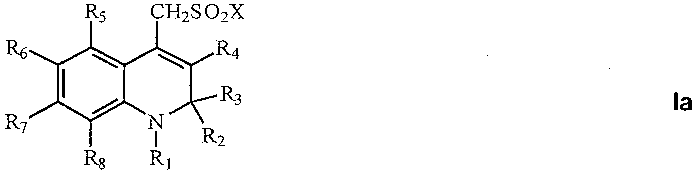 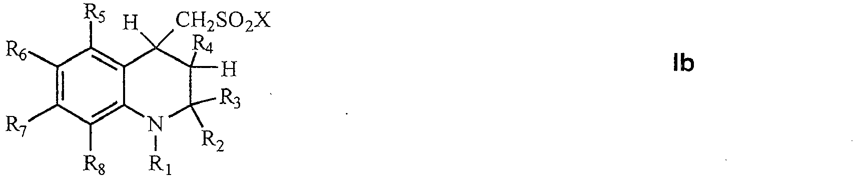 worin R₁ Wasserstoff oder eine Kohlenwasserstoffgruppe mit 1-20 C-Atomen bedeutet, wobei die Kohlenwasserstoffgruppe gegebenenfalls ein oder mehrere Heteroatome oder/und einen oder mehrere Substituenten enthalten kann,
   R₂, R₃, R₄, R₅, R₆, R₇ und R₈ bei jedem Auftreten unabhängig voneinander Wasserstoff, Halogen, eine Hydroxy-, Amino-, Sulfo-, Carboxy- oder Aldehydgruppe oder eine Kohlenwasserstoffgruppe mit 1-20 C-Atomen bedeutet, wobei die Kohlenwasserstoffgruppe gegebenenfalls ein oder mehrere Heteroatome oder/und einen oder mehrere Substituenten enthalten kann, oder die Reste R₁ und R₈ zusammen ein Ringsystem bilden und
   X OH, Halogen, -O-R₉, -S-R₁₀ oder -NR₁₁R₁₂ bedeutet, wobei R₉, R₁₀, R₁₁ und R₁₂ jeweils unabhängig voneinander Wasserstoff oder einen C1 bis C20 Kohlenwasserstoffrest bedeuten, welcher gegebenenfalls ein oder mehrere Heteroatome oder/und einen oder mehrere Substituenten, insbesondere -SO₃H, -PO₃H₂ und -COOH enthalten kann.
Punkt 7: Verbindung nach Punkt 6,
   worin R₁ einen Aryl- oder Alkylrest, insbesondere einen C5 bis C15-Aryl- oder einen C1 bis C20-Alkylrest darstellt, R₂ und R₃ Methyl sind und R₄ Wasserstoff bedeutet.
Punkt 8:
   Verbindung nach Punkt 6 oder 7, **dadurch gekennzeichnet,**
   dass R₇ einen Hydroxy- oder Methoxyrest darstellt.
Punkt 9:
   Verfahren nach einem der Punkte 6 bis 8,
   **dadurch gekennzeichnet,**
   dass R₆ eine Nitrosogruppe darstellt.
Punkt 10:
   Verfahren nach einem der Punkte 6 bis 7,
   **dadurch gekennzeichnet,**
   dass R₆ eine Formyl- oder eine Hydroxymethylgruppe darstellt.
Punkt 11:
   Verbindung nach einem der Punkte 6 bis 10,
   **dadurch gekennzeichnet,**
   dass X Halogen, insbesondere Cl bedeutet.
Punkt 12:
   Verbindung nach einem der Punkte 6 bis 11,
   **dadurch gekennzeichnet,**
   dass X den Rest -NR₁₁R₁₂ darstellt, wobei die Reste R₁₁ und R₁₂ wie in Punkt 6 definiert sind.
Punkt 13:
   Verfahren zur Herstellung von -SO₂X enthaltenden Farbstoffen der allgemeinen Formeln II bis VII 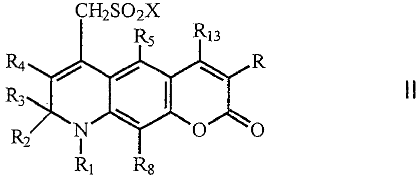 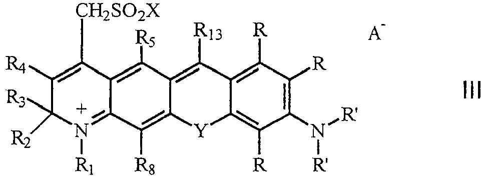 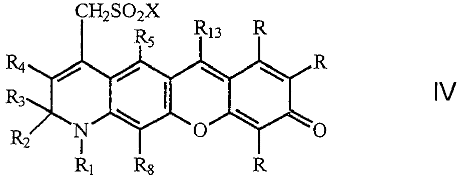 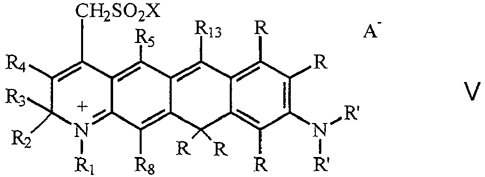 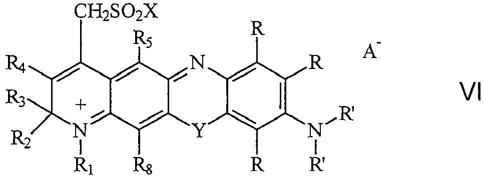 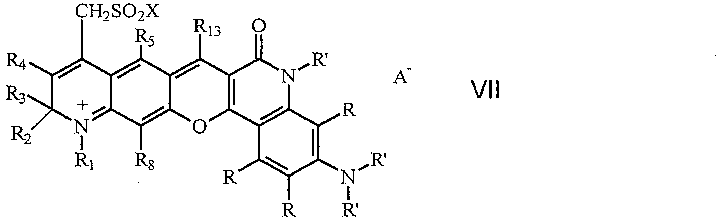 worin R₁, R₂, R₃, R₄. R₅ und R₈ wie in den Punkten 1 bis 12 definiert sind, R bei jedem Auftreten gleich oder verschieden sein kann und wie R₁, R₂, R₃, R₄. R₅ und R₈ definiert ist und R' bei jedem Auftreten unabhängig voneinander Wasserstoff oder eine Kohlenwasserstoffgruppe mit 1-20 C-Atomen bedeutet, wobei die Kohlenwasserstoffgruppe gegebenenfalls ein oder mehrere Heteroatome oder/und einen oder mehrere Substituenten enthalten kann, oder die Reste R und R' zusammen ein Ringsystem bilden, das eine oder mehrere Doppelbindungen enthalten kann,
   R₁₃ bei jedem Auftreten unabhängig voneinander Wasserstoff oder eine Kohlenwasserstoffgruppe mit 1-20 C-Atomen bedeutet, wobei die Kohlenwasserstoffgruppe gegebenenfalls ein oder mehrere Heteroatome oder/und einen oder mehrere Substituenten enthalten kann, wobei R₁₃ insbesondere Wasserstoff, Aryl, Carboxyphenyl, Alkyl, Perfluoralkyl, Cycloalkyl, Pyridyl oder Carboxypyridyl darstellt,
   X OH, Halogen, -O-R₉, -S-R₁₀ oder -NR₁₁R₁₂ bedeutet, wobei R₉, R₁₀, R₁₁ und R₁₂ jeweils unabhängig voneinander Wasserstoff oder einen C1 bis C20 Kohlenwasserstoffrest bedeuten, welcher gegebenenfalls ein oder mehrere Heteroatome oder einen oder mehrere Substituenten enthalten kann, und
   Y in Formel III O, S oder Se sowie Y in Formel VI O, S oder C(R)₂ bedeutet
   **dadurch gekennzeichnet,**
   dass man entsprechende Verbindungen der Formeln II' bis VII' 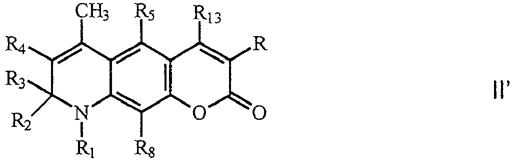 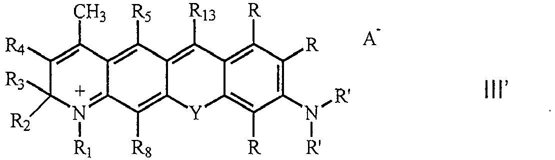 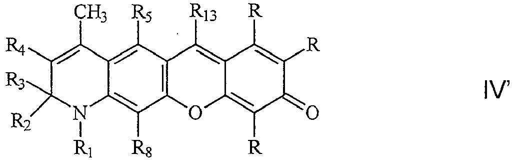 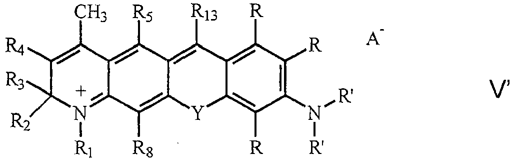 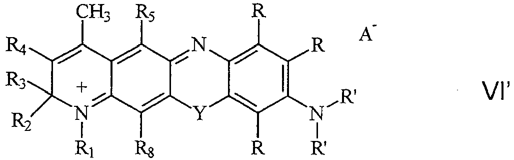 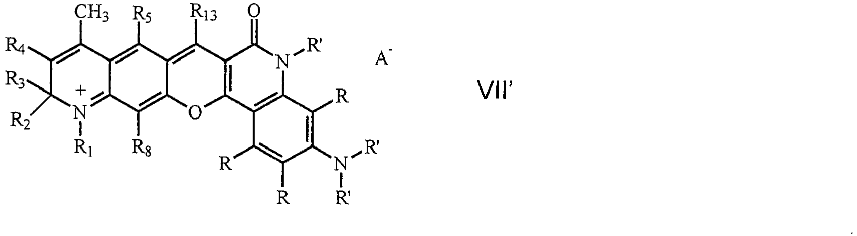 sulfoniert, mit der Maßgabe, dass für Verbindungen der Formel III mit Y = O und Verbindungen der Formel IV X nicht OH bedeutet.
Punkt 14:
   Verwendung einer Verbindung nach einem der Punkte 6 bis 12 oder erhältlich gemäß dem Verfahren nach einem der Punkte 1 bis 5 zur Herstellung von polycyclischen Farbstoffen.
Punkt 15:
   Verwendung nach Punkt 14 zur Herstellung von polycyclischen Farbstoffen der Formeln II bis VII.
Punkt 16:
   Verfahren zur Herstellung polycyclischer Farbstoffe,
   **dadurch gekennzeichnet,**
   dass man Verbindungen, welche eine Dihydrochinolinendgruppe mit einer 4-Methylgruppe aufweisen, sulfoniert, - gegebenenfalls zum Tetrahydrochinolin hydriert - mit der Maßgabe, dass der polycyclische Farbstoff keine Verbindung der Formel III mit Y = O und X = OH oder der Formel IV mit X = OH ist.
Punkt 17:
   Polycyclischer Farbstoff erhältlich gemäß dem Verfahren nach einem der Punkte 13 bis 16.
Punkt 18:
   Polycyclischer Farbstoff der allgemeinen Formel II bis VII 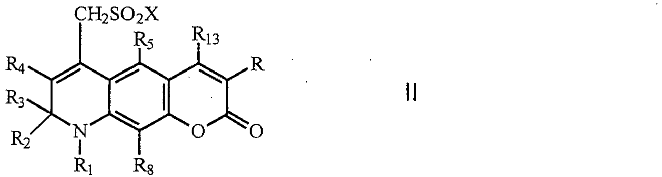 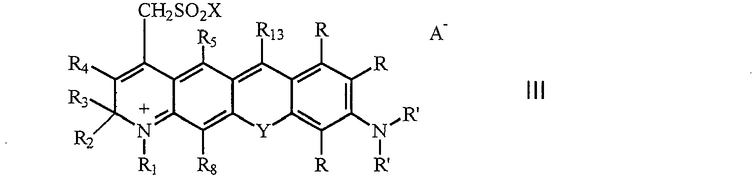 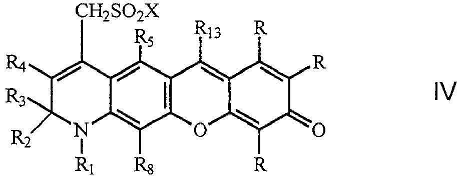 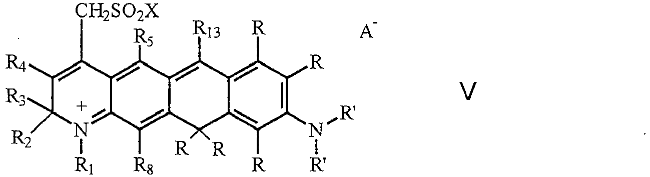 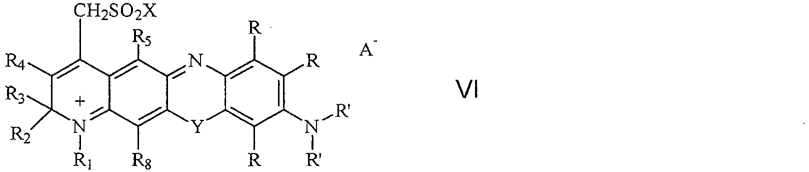 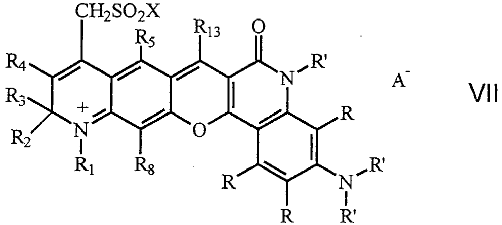 worin
   R' Wasserstoff oder eine Kohlenwasserstoffgruppe mit 1-20 C-Atomen bedeutet, wobei die Kohlenwasserstoffgruppe gegebenenfalls ein oder mehrere Heteroatome oder/und einen oder mehrere Substituenten enthalten kann,
   R bei jedem Auftreten unabhängig voneinander Wasserstoff, Halogen, eine Hydroxy-, Amino-, Sulfo-, Carboxy- oder Aldehydgruppe oder eine Kohlenwasserstoffgruppe mit 1-20 C-Atomen bedeutet, wobei die Kohlenwasserstoffgruppe gegebenenfalls ein oder mehrere Heteroatome oder/und einen oder mehrere Substituenten enthalten kann, oder die Reste R' und R zusammen ein Ringsystem bilden, das eine oder mehrere Mehrfachbindungen enthalten kann,
   R₁₃ bei jedem Auftreten unabhängig voneinander Wasserstoff oder eine Kohlenwasserstoffgruppe mit 1-20 C-Atomen bedeutet, wobei die Kohlenwasserstoffgruppe gegebenenfalls ein oder mehrere Heteroatome oder/und einen oder mehrere Substituenten enthalten kann, wobei R₁₃ insbesondere Wasserstoff, Aryl, Carboxyphenyl, Alkyl, Perfluoralkyl, Cycloalkyl, Pyridyl oder Carboxypyridyl darstellt,
   X OH, Halogen, -O-R₉, -S-R₁₀ oder -NR₁₁R₁₂ bedeutet, wobei R₉, R₁₀, R₁₁ und R₁₂ jeweils unabhängig voneinander Wasserstoff oder einen C1 bis C20 Kohlenwasserstoffrest bedeuten, welcher gegebenenfalls ein oder mehrere Heteroatome oder einen oder mehrere Substituenten enthalten kann, und
   Y in Formel III O, S, oder Se sowie Y in Formel VI O, S oder C(R)₂ darstellt,
   mit der Maßgabe, dass der Farbstoff keine Verbindung der allgemeinen Formel III mit Y = O und X = OH oder der allgemeinen Formel IV mit X = OH ist.
Punkt 19:
   Polycyclischer Farbstoff nach Punkt 17 oder 18,
   **dadurch gekennzeichnet,**
   dass X Halogen, insbesondere CI, bedeutet.
Punkt 20:
   Polycyclischer Farbstoff nach Punkt 17 oder 18,
   **dadurch gekennzeichnet,**
   dass X für den Rest -NR₁₁R₁₂ steht, wobei die Reste R₁₁ und R₁₂ wie in Punkt 18 definiert sind.
Punkt 21:
   Polycyclischer Farbstoff nach Punkt 20,
   **dadurch gekennzeichnet,**
   dass R₁₁ oder/und R₁₂ einen mit -COOH substituierten Alkyl- oder Arylrest darstellt.
Punkt 22:
   Verwendung eines Farbstoffs nach einem der Punkte 17 bis 21 zur Markierung eines Analyten.
Punkt 23:
   Verwendung nach Punkt 22,
   **dadurch gekennzeichnet,**
   dass es sich bei dem Analyten um ein Biomolekül, insbesondere um ein Peptid oder ein Nukleotid, handelt.
Punkt 24:
   Verwendung nach Punkt 22 oder 23,
   **dadurch gekennzeichnet,**
   dass die Markierung durch Bindung des Farbstoffs an eine NH₂- oder SH-Gruppe des Analyten erfolgt.
Punkt 25:
   Verwendung eines Farbstoffs nach Punkt 19 zur Markierung eines Analyten, wobei die Bindung des Farbstoffs durch Kopplung an eine Aminogruppe des Analyten erfolgt.
Punkt 26:
   Verwendung eines Farbstoffs nach Punkt 21 zur Markierung eines Analyten, wobei die Bindung des - z.B. als NHS-Ester aktivierten - Farbstoffs durch Kopplung an eine Aminogruppe des Analyten erfolgt.
Punkt 27:
   Verwendung eines Farbstoffes nach einem der Punkte 17 bis 21 zur Kopplung an einen weiteren Farbstoff.
**Punkt 28:**
   Verwendung nach Punkt 27,
   **dadurch gekennzeichnet,**
   dass die Kopplung über eine Aminogruppe des weiteren Farbstoffs erfolgt und dadurch ein FRET-Pärchen gebildet wird.

## Patentansprüche

1. Polycyclischer Farbstoff der allgemeinen Formel II", V" - VII" 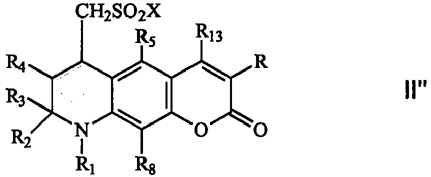 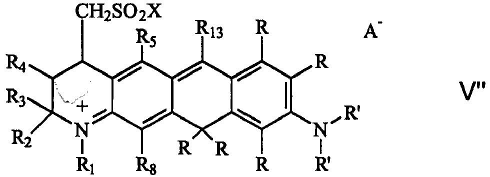 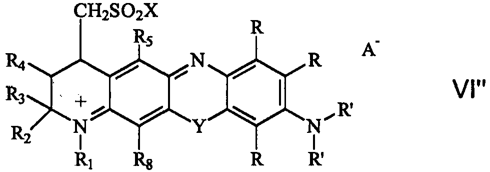 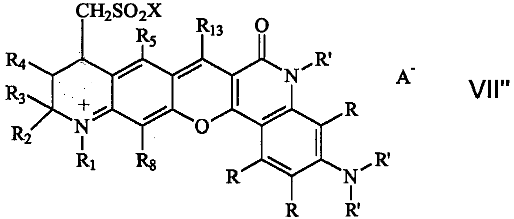 worin
R₁ Wasserstoff oder eine Kohlenwasserstoffgruppe mit 1-20 C-Atomen bedeutet, wobei die Kohlenwasserstoffgruppe gegebenenfalls ein oder mehrere Heteroatome oder/und einen oder mehrere Substituenten enthalten kann,
R₂, R₃, R₄, R₅, R₆, R₇ und R₈ bei jedem Auftreten unabhängig voneinander Wasserstoff, Halogen, eine Hydroxy-, Amino-, Sulfo-, Carboxy- oder Aldehydgruppe oder eine Kohlenwasserstoffgruppe mit 1-20 C-Atomen bedeutet, wobei die Kohlenwasserstoffgruppe gegebenenfalls ein oder mehrere Heteroatome oder/und einen oder mehrere Substituenten enthalten kann, oder die Reste R₁ und R₈ zusammen ein Ringsystem bilden,
R' Wasserstoff oder eine Kohlenwasserstoffgruppe mit 1-20 C-Atomen bedeutet, wobei die Kohlenwasserstoffgruppe gegebenenfalls ein oder mehrere Heteroatome oder/und einen oder mehrere Substituenten enthalten kann,
R bei jedem Auftreten unabhängig voneinander Wasserstoff, Halogen, eine Hydroxy-, Amino-, Sulfo-, Carboxy- oder Aldehydgruppe oder eine Kohlenwasserstoffgruppe mit 1-20 C-Atomen bedeutet, wobei die Kohlenwasserstoffgruppe gegebenenfalls ein oder mehrere Heteroatome oder/und einen oder mehrere Substituenten enthalten kann, oder die Reste R' und R zusammen ein Ringsystem bilden, das eine oder mehrere Mehrfachbindungen enthalten kann,
R₁₃ bei jedem Auftreten unabhängig voneinander Wasserstoff oder eine Kohlenwasserstoffgruppe mit 1-20 C-Atomen bedeutet, wobei die Kohlenwasserstoffgruppe gegebenenfalls ein oder mehrere Heteroatome oder/und einen oder mehrere Substituenten enthalten kann, wobei R₁₃ insbesondere Wasserstoff, Aryl, Carboxyphenyl, Alkyl, Perfluoralkyl, Cycloalkyl, Pyridyl oder Carboxypyridyl darstellt,
X OH, Halogen, -O-R₉, -S-R₁₀ oder -NR₁₁R₁₂ bedeutet, wobei R₉, R₁₀, R₁₁ und R₁₂ jeweils unabhängig voneinander Wasserstoff oder einen C1 bis C20 Kohlenwasserstoffrest bedeuten, welcher gegebenenfalls ein oder mehrere Heteroatome oder einen oder mehrere Substituenten enthalten kann,
Y in Formel VI" O, S oder C(R)₂ darstellt, und
A- ein Anion bedeutet.

2. Polycyclischer Farbstoff nach Anspruch 1,
**dadurch gekennzeichnet,**
**dass** X Halogen, insbesondere CI, bedeutet.

3. Polycyclischer Farbstoff nach Anspruch 1,
**dadurch gekennzeichnet,**
**dass** X für den Rest -NR₁₁R₁₂ steht, wobei die Reste R₁₁ und R₁₂ wie in Anspruch 1 definiert sind.

4. Polycyclischer Farbstoff nach Anspruch 3,
**dadurch gekennzeichnet,**
**dass** R₁₁ oder/und R₁₂ einen mit -COOH substituierten Alkyl- oder Arylrest darstellt.

5. Polycyclischer Farbstoff nach Anspruch 1,
**dadurch gekennzeichnet,**
**dass** er aus der Gruppe bestehend aus 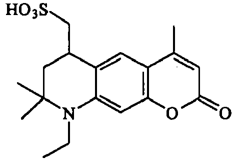 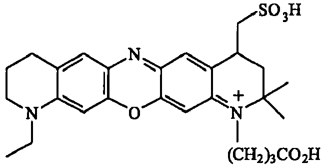 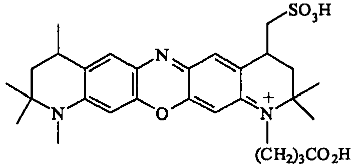 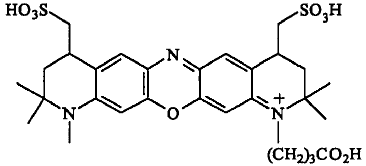 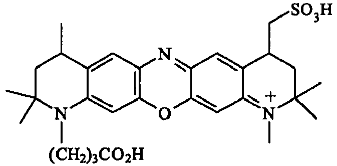 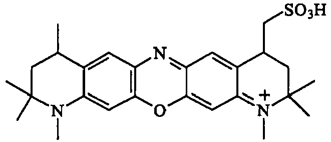 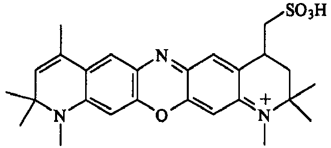 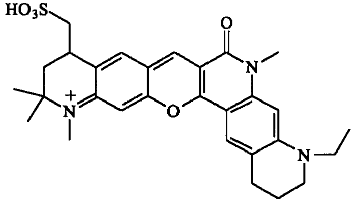 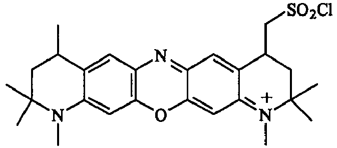 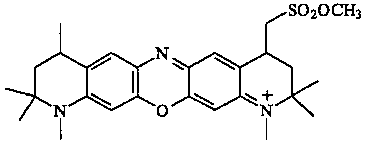 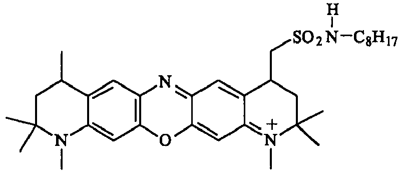 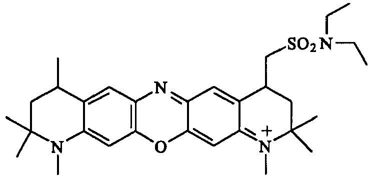 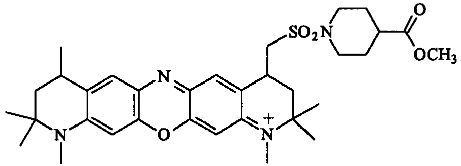 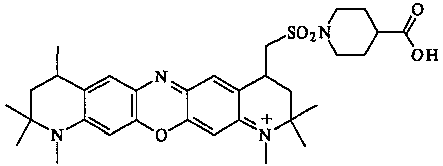 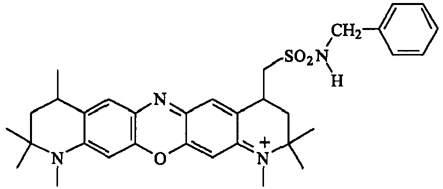 ausgewählt ist.

6. Verfahren zur Herstellung von polycyclischen Farbstoffen nach einem der Ansprüche 1 bis 5,
**dadurch gekennzeichnet,**
**dass** man Verbindungen der Formeln II', V' - VII' 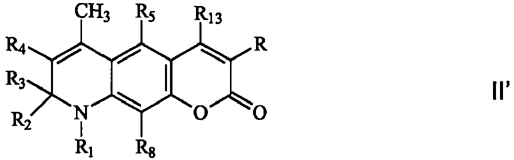 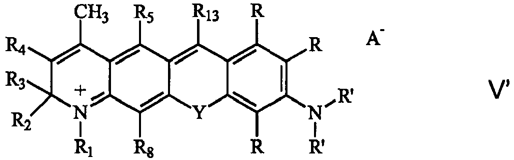 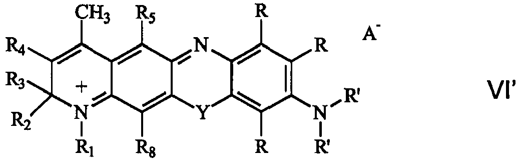 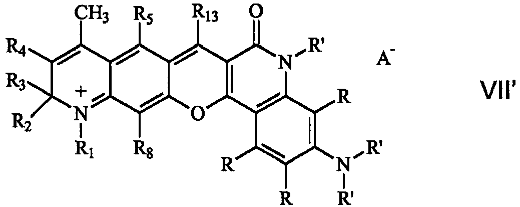 zu entsprechenden Verbindungen der allgemeinen Formeln II, V - VII 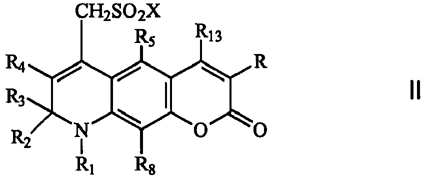 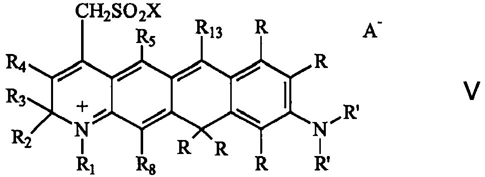 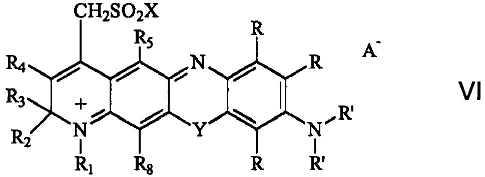 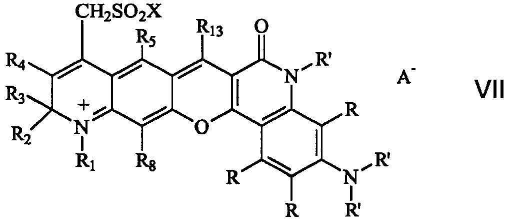 sulfoniert, und die Verbindungen der allgemeinen Formeln II, V - VII anschließend zu entsprechenden Tetrahydrochinolinen der allgemeinen Formeln II", V" - VII" hydriert.

7. Verwendung eines Farbstoffs nach einem der Ansprüche 1 bis 5 zur Markierung eines Analyten.

8. Verwendung nach Anspruch 7,
**dadurch gekennzeichnet,**
**dass** es sich bei dem Analyten um ein Biomolekül, insbesondere um ein Peptid oder ein Nukleotid, handelt.

9. Verwendung nach Anspruch 7 oder 8,
**dadurch gekennzeichnet,**
**dass** die Markierung durch Bindung des Farbstoffs an eine NH₂- oder SH-Gruppe des Analyten erfolgt.

10. Verwendung eines Farbstoffes nach einem der Ansprüche 1 bis 5 zur Kopplung an einen weiteren Farbstoff.

11. Verwendung nach Anspruch 10,
**dadurch gekennzeichnet,**
**dass** die Kopplung über eine Aminogruppe des weiteren Farbstoffs erfolgt und dadurch ein FRET-Pärchen gebildet wird.

## Claims

1. Polycyclic dye of general formula II", V" to VII" 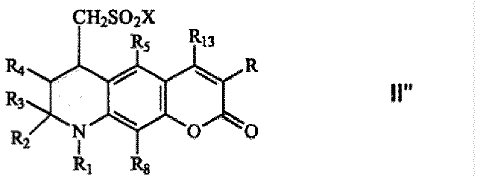 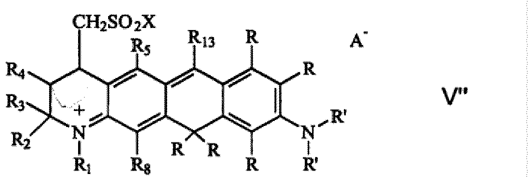 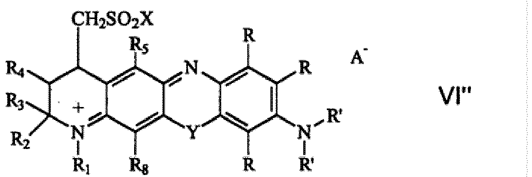 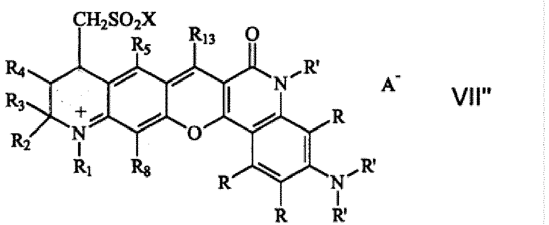 in which
R₁ is hydrogen or a hydrocarbon group having 1-20 C atoms, wherein the hydrocarbon group may optionally contain one or more heteroatoms and/or one or more substituents,
R₂, R₃, R₄, R₅, R₆, R₇ and R₈ are, mutually independently on each occurrence, hydrogen, halogen, a hydroxy, amino, sulfo, carboxy or aldehyde group or a hydrocarbon group having 1-20 C atoms, wherein the hydrocarbon group may optionally contain one or more heteroatoms and/or one or more substituents, or the residues R₁ and R₈ together form a ring system,
R' is hydrogen or a hydrocarbon group having 1-20 C atoms, wherein the hydrocarbon group may optionally contain one or more heteroatoms and/or one or more substituents,
R is, mutually independently on each occurrence, hydrogen, halogen, a hydroxy, amino, sulfo, carboxy or aldehyde group or a hydrocarbon group having 1-20 C atoms, wherein the hydrocarbon group may optionally contain one or more heteroatoms and/or one or more substituents, or the residues R' and R together form a ring system which may contain one or more multiple bonds,
R₁₃ is, mutually independently on each occurrence, hydrogen or a hydrocarbon group having 1-20 C atoms, wherein the hydrocarbon group may optionally contain one or more heteroatoms and/or one or more substituents, wherein R₁₃ in particular represents hydrogen, aryl, carboxyphenyl, alkyl, perfluoroalkyl, cycloalkyl, pyridyl or carboxypyridyl,
X is OH, halogen, -O-R₉, -S-R₁₀ or -NR₁₁R₁₂, wherein R₉, R₁₀, R₁₁ and R₁₂ are, mutually independently in each case, hydrogen or a C1 to C20 hydrocarbon residue, which may optionally contain one or more heteroatoms or one or more substituents, Y in formula VI" represents O, S or C(R)₂, and
A⁻ is an anion.

2. Polycyclic dye according to claim 1, **characterised in that** X is halogen, in particular CI.

3. Polycyclic dye according to claim 1, **characterised in that** X denotes the residue -NR₁₁R₁₂, wherein the residues R₁₁ and R₁₂ are as defined in claim 1.

4. Polycyclic dye according to claim 3, **characterised in that** R₁₁ and/or R₁₂ represent an alkyl or aryl residue substituted with -COOH.

5. Polycyclic dye according to claim 1, **characterised in that** it is selected from the group consisting of

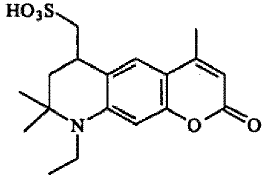

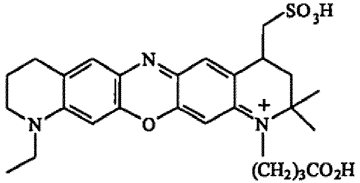

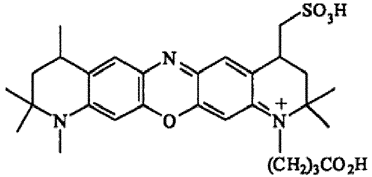

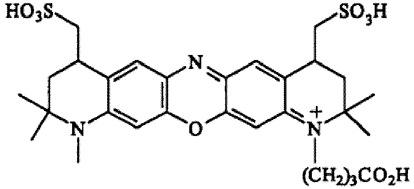

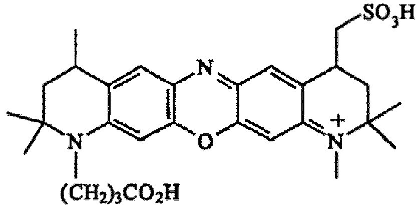

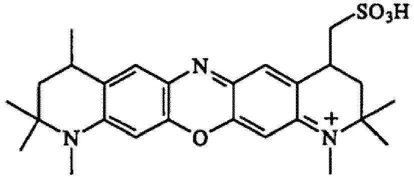

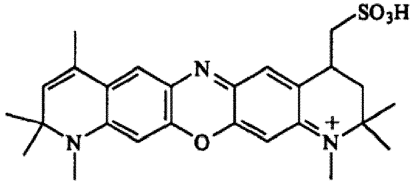

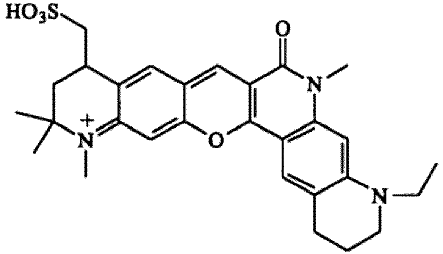

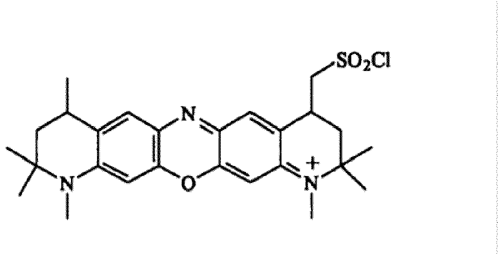

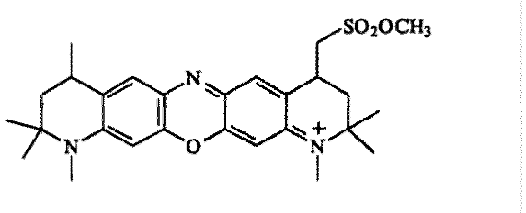

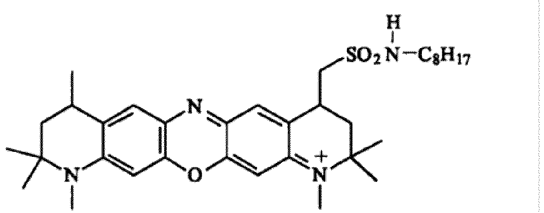

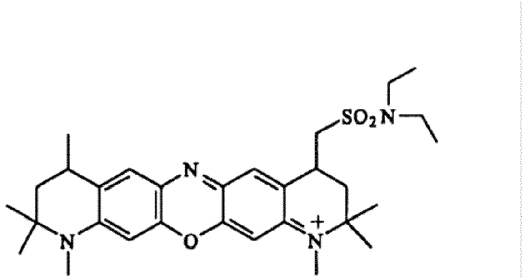

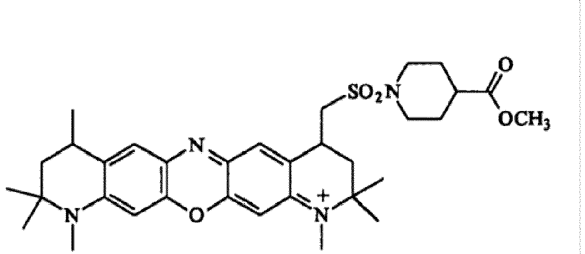

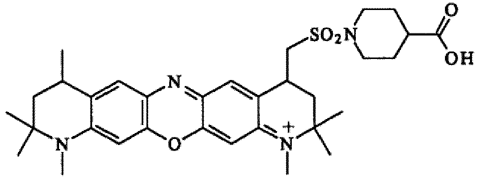

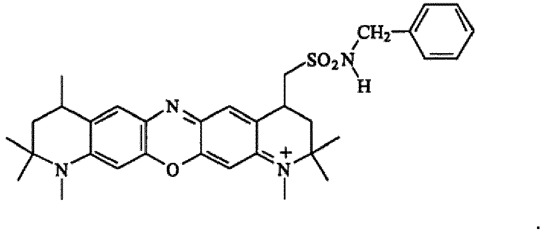

6. Method for producing polycyclic dyes according to any of claims 1 to 5, **characterised in that** compounds of formulae II', V' to VII' 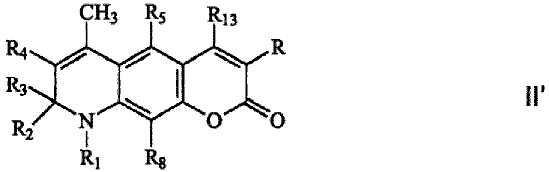 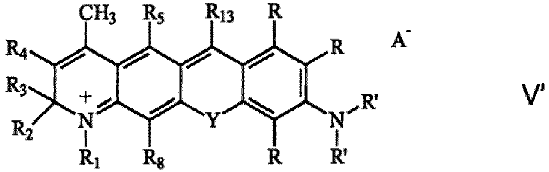 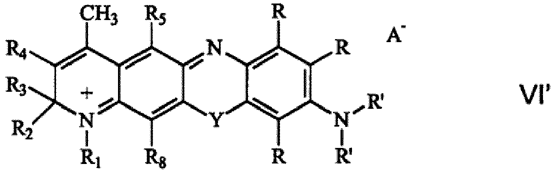 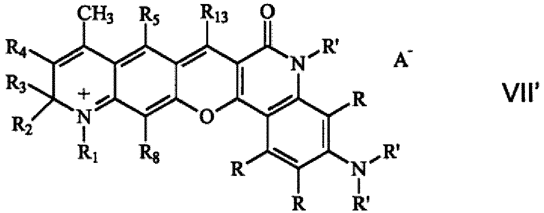 are sulphonated to form corresponding compounds of general formulae II, V to VII 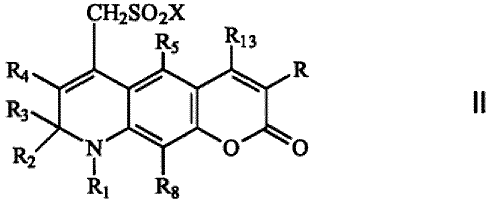 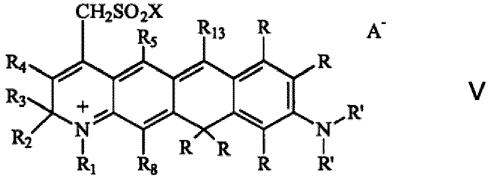 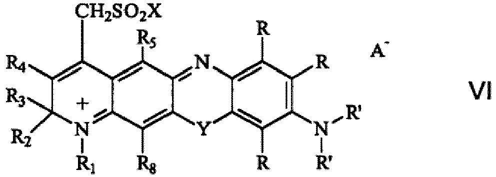 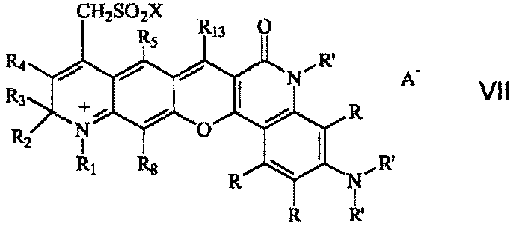 and the compounds of general formulae II, V to VII are then hydrogenated to form corresponding tetrahydroquinolines of general formulae II", V" to VII".

7. Use of a dye according to any of claims 1 to 5 for labelling an analyte.

8. Use according to claim 7, **characterised in that** the analyte is a biomolecule, in particular a peptide or a nucleotide.

9. Use according to either claim 7 or claim 8, **characterised in that** labelling takes place by binding the dye to an NH₂ or SH group of the analyte.

10. Use of a dye according to any of claims 1 to 5 for coupling to a further dye.

11. Use according to claim 10, **characterised in that** coupling takes place via an amino group of the further dye and, in this manner, a FRET pair is formed.

## Revendications

1. Colorant polycyclique de formules générales II", V" à VII" 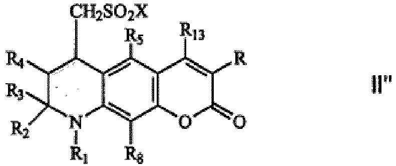 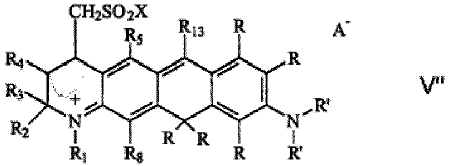 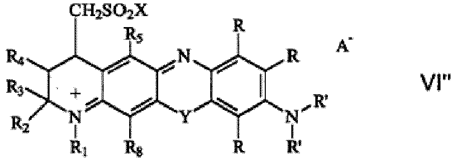 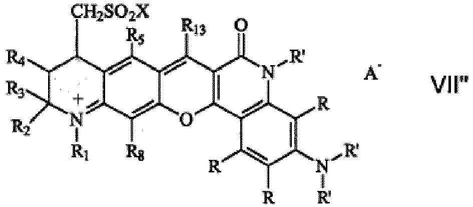 dans lesquelles
R₁ désigne un atome d'hydrogène ou un groupe hydrocarbure comportant 1 à 20 atomes de carbone, le groupe hydrocarbure pouvant contenir éventuellement un ou plusieurs hétéroatomes et/ou un ou plusieurs substituants,
R₂, R₃, R₄, R₅, R₆, R₇ et R₈ désignant à chaque occurrence, indépendamment les unes des autres, un atome d'hydrogène, d'halogène, un groupe hydroxy, amino, sulfo, carboxy ou aldéhyde ou un groupe hydrocarbure comportant 1 à 20 atomes de carbone, le groupe hydrocarbure pouvant contenir éventuellement un ou plusieurs hétéroatomes et/ou un ou plusieurs substituants, ou les radicaux R₁ et R₈ forment conjointement un système cyclique,
R' désigne un atome d'hydrogène ou un groupe hydrocarbure comportant 1 à 20 atomes de carbone, le groupe hydrocarbure pouvant contenir éventuellement un ou plusieurs hétéroatomes et/ou un ou plusieurs substituants,
R désigne à chaque occurrence, indépendamment les unes des autres, un atome d'hydrogène, d'halogène, un groupe hydroxy, amino, sulfo, carboxy ou aldéhyde ou un groupe hydrocarbure comportant 1 à 20 atomes de carbone, le groupe hydrocarbure pouvant contenir éventuellement un ou plusieurs hétéroatomes et/ou un ou plusieurs substituants, ou les radicaux R' et R forment conjointement un système cyclique, qui peut renfermer une ou plusieurs liaisons multiples,
R₁₃ désigne à chaque occurrence, indépendamment les unes des autres, un atome d'hydrogène ou un groupe hydrocarbure comportant 1 à 20 atomes de carbone, le groupe hydrocarbure pouvant contenir éventuellement un ou plusieurs hétéroatomes et/ou un ou plusieurs substituants, R₁₃ représentant en particulier un atome d'hydrogène, un groupe aryle, carboxyphényle, alkyle, perfluoroalkyle, cycloalkyle, pyridyle ou carboxypyridyle,
X désigne OH, un atome d'halogène, -O-R₉, -S-R₁₀ ou -NR₁₁R₁₂, où R₉, R₁₀, R₁₁ et R₁₂ désignent, indépendamment les uns des autres, un atome d'hydrogène ou un radical hydrocarbure en C₁ à C₂₀ qui peut contenir éventuellement un ou plusieurs hétéroatomes ou un ou plusieurs substituants,
Y désigne, dans la formule VI", O, S ou C(R)₂, et
A⁻ désigne un anion.

2. Colorant polycyclique selon la revendication 1,
**caractérisé en ce que**
X représente un atome d'halogène, en particulier Cl.

3. Colorant polycyclique selon la revendication 1,
**caractérisé en ce que**
X représente le radical -NR₁₁R₁₂, les radicaux R₁₁ et R₁₂ étant tels que définis dans la revendication 1.

4. Colorant polycyclique selon la revendication 3,
**caractérisé en ce que**
R₁₁ et/ou R₁₂ représentent un radical alkyle ou aryle substitué par -COOH.

5. Colorant polycyclique selon la revendication 1,
**caractérisé en ce que**
il est choisi dans le groupe constitué par

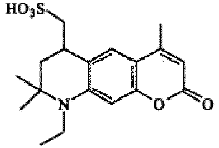

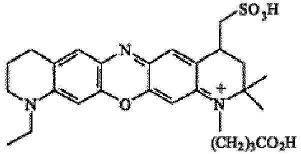

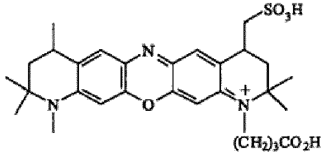

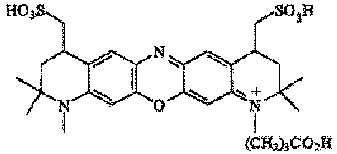

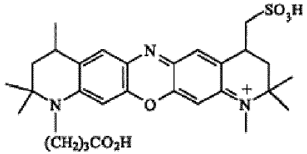

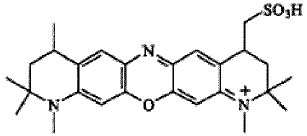

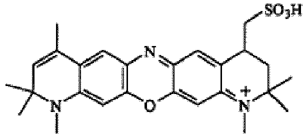

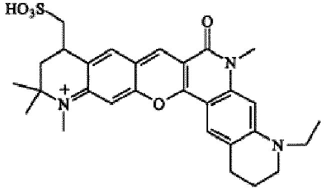

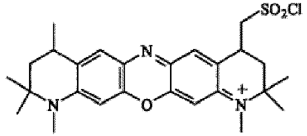

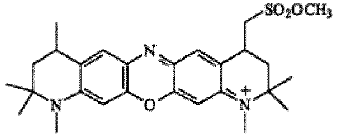

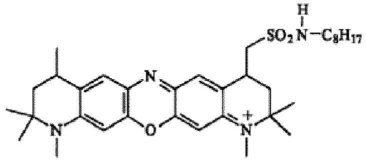

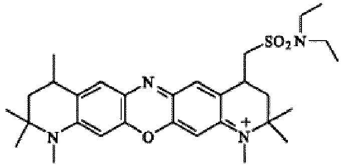

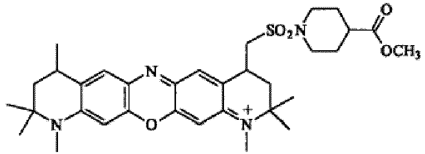

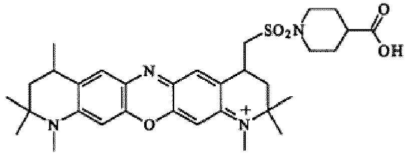

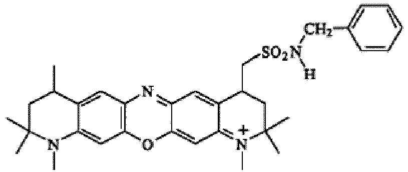

6. Procédé de production de colorants polycycliques selon l'une des revendications 1 à 5,
**caractérisé en ce que**
l'on sulfone les composés de formules II', V' à VII' 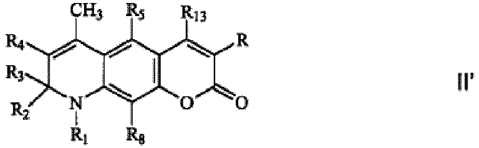 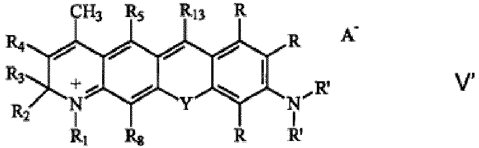 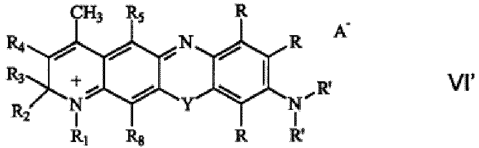 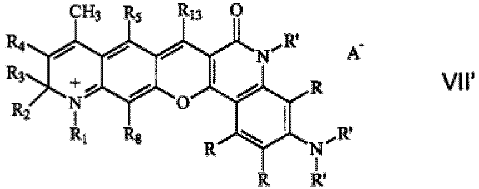 pour donner les composés correspondants de formules générales II, V à VII 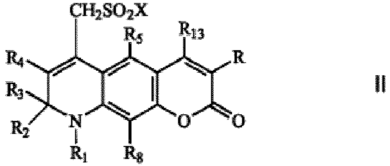 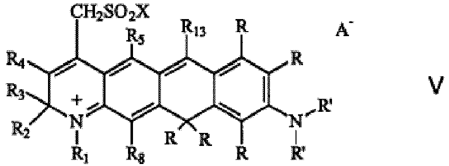 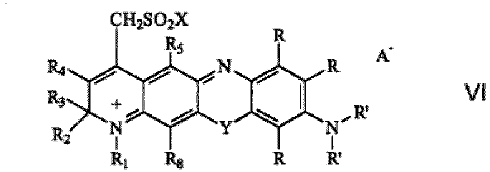 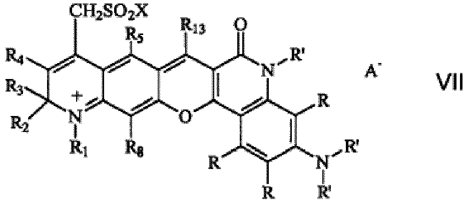 5 et **en ce que** l'on convertit ensuite par hydrogénation les composés de formules générales II, V à VII en les tétrahydroquinolines correspondantes de formules générales II", V" à VII".

7. Utilisation d'un colorant selon l'une des revendications 1 à 5, pour le marquage d'un analyte.

8. Utilisation selon la revendication 7,
**caractérisée en ce que**
l'analyte est une biomolécule, en particulier un peptide ou un nucléotide.

9. Utilisation selon la revendication 7 ou 8,
**caractérisée en ce que**
le marquage s'effectue par liaison du colorant à un groupe NH₂- ou SH- de l'analyte.

10. Utilisation d'un colorant selon l'une des revendications 1 à 5, pour le couplage à un autre colorant.

11. Utilisation selon la revendication 10,
**caractérisée en ce que**
le couplage s'effectue par un groupe amino de l'autre colorant et ainsi est formée une paire de FRET.
